**Europäisches Patentamt**

**European Patent Office**

**Office européen des brevets**

(11) Veröffentlichungsnummer : **0 505 321 A2**

# (12) EUROPÄISCHE PATENTANMELDUNG

(21) Anmeldenummer : **92810182.3**

(22) Anmeldetag : **12.03.92**

(51) Int. Cl.$^5$ : **A61M 15/00**

(30) Priorität : **21.03.91 EP 91810195**
**28.08.91 CH 2515/91**

(43) Veröffentlichungstag der Anmeldung :
**23.09.92 Patentblatt 92/39**

(84) Benannte Vertragsstaaten :
**AT BE CH DE DK ES FR GB GR IT LI LU NL PT SE**

(71) Anmelder : **CIBA-GEIGY AG**
**Klybeckstrasse 141**
**CH-4002 Basel (CH)**

(72) Erfinder : **Altermatt, Daniel, Dr.**
**Neumattstrasse 37**
**CH-4142 Münchenstein (CH)**
Erfinder : **Hilpert, Hanspeter**
**Untere Dorfstrasse 5**
**W-7888 Rheinfelden/Nollingen (DE)**
Erfinder : **Khanna, Satish Chandra, Dr.**
**Rütistrasse 26**
**CH-4103 Bottmingen (CH)**
Erfinder : **Dubach, Werner F.**
**Hubrainstrasse 4**
**CH-8124 Maur/ZH (CH)**
Erfinder : **Spaltenstein, Anton**
**Baltenswilerstrassse 28**
**CH-8303 Bassersdorf/ZH (CH)**

(54) **Inhalator.**

(57)   Ein Inhalator zum Einbringen einer dosierten Menge von Festkörpern, zum Beispiel pharmazeutisch wirksamen Pulvern, in einen von einem Benutzer angesaugten Luftstrom umfasst eine Vorratskammer (4), in die ein Dosierstift (5) hineinragt. Der Dosierstift (5) ist mit einer Dosierkerbe (50) versehen, welche die Menge an Festkörpern festlegt, die dem Lufstrom beigemischt wird. Die Vorratskammer (4) und der Dosierstift (5) sind relativ zueinander so bewegbar, dass sich in einer ersten Relativposition von Dosierstift (5) und Vorratskammer (4) die Dosierkerbe (50) des Dosierstifts (5) in der Vorratskammer (4) befindet, wo sie mit Festkörpern befüllt wird, und in einer zweiten Relativstellung sich im Luftkanal befindet, wo die Festkörper dem Luftstrom beigemischt werden. Die axiale Relativbewegung kann dabei durch eine Verdrehung zweier Gehäuseteile (101, 102) über Schrägflächen (103) bewirkt werden.

Fig. 14

EP 0 505 321 A2

Jouve, 18, rue Saint-Denis, 75001 PARIS

Die Erfindung betrifft einen Inhalator zum Einbringen einer dosierten Menge von Festkörpern in einen von einem Benutzer angesaugten Luftstrom gemäss Oberbegriff von Patentanspruch 1.

Festkörperinhalatoren sind heutzutage in grosser Zahl und in vielen Ausführungsformen erhältlich. Mit diesen Inhalatoren werden vornehmlich pharmazeutisch wirksame Stoffe oder Stoffgemische, insbesondere pulverförmige Stoffe oder Stoffgemische, von einem Benutzer inhaliert. Ein solcher Inhalator ist beispielsweise aus der CH-A-666,823 bekannt. Der dort beschriebene Inhalator umfasst eine Vorratskammer für die Festkörper sowie einen Luftkanal, der eine Lufteinlassöffnung für die angesaugte Luft mit einem Auslass für das im Inhalator erzeugte Festkörper-Luft-Gemisch verbindet. Zur Festlegung der Festkörpermenge und zum Einbringen dieser festgelegten Festkörpermenge in den Luftstrom ist ein mit einer Dosierkerbe versehener Dosierstift vorgesehen, der um seine Längsachse drehbar ausgebildet ist. Die Dosierkerbe des Dosierstifts nimmt in einer ersten Stellung die durch das Volumen der Dosierkerbe festgelegte Menge an Festkörpern auf. Anschliessend wird der Dosierstift um 180° um seine Achse in eine zweite Stellung gedreht, in der die Festkörper aus der Dosierkerbe herausfallen können und so in den Luftkanal gelangen, wo sie dem Luftstrom beigemischt werden.

Der beschriebene Inhalator hat verschiedene Nachteile. So ist es erforderlich, dass die Festkörper gut fliessfähig sind, damit gewährleistet ist, dass die Dosierkerbe stets schnell, zuverlässig und vollständig befüllt wird. Da Granulate und sonstige gröbere Festkörper für die Inhalation aber ungeeignet sind, liegen die pharmazeutisch wirksamen Stoffe oder Stoffgemische, z.B. antiasthmatisch wirksame Stoffe oder Stoffgemische, oft als sehr feinkörniges Pulver vor. Diese feinkörnigen Pulver haben aber zumeist die sehr nachteilige Eigenschaft, dass sie nicht oder nur sehr schlecht liessfähig sind. Das kann aber wiederum zur Folge haben, dass die Dosierkerbe nicht vollständig befüllt wird und damit auch die dosierte Festkörpermenge schwankt. Gerade aber ein in Atemnot (z.B. durch einen Asthmaanfall bedingt) geratener Benutzer muss aber schnell und zuverlässig eine bestimmte Menge an Pulver inhalieren können, um eine schnelle Linderung zu erfahren.

Ein weiterer Nachteil des beschriebenen Inhalators ist, dass das Einbringen der Festkörper in den Luftkanal mit Hilfe eines drehbaren Dosierstifts erfolgt. So kann es vorkommen, dass Festkörper in die Hohlräume (Lagerung) um den drehbaren Dosierstift herum eindringen können und bewirken, dass der Dosierstift entweder nur noch sehr schwer drehbar ist oder sich sogar gänzlich "festfrisst", was insbesondere für asthmaanfällige Benutzer schwerwiegende Folgen haben kann, da dann in einem Notfall das Gerät nicht funktionstüchtig sein kann.

Weiterhin nachteilig bei dem beschriebenen Inhalator ist auch die Tatsache, dass die feinkörnigen Pulver in der Vorratskammer nicht in einem gesonderten Vorratsbehälter, z.B. einer Kapsel gelagert werden können. Das ist insbesondere für solche Pulver nachteilig, deren pharmazeutische Wirkung bei einer Lagerung im Dauerkontakt mit der Luft nachlässt. Ausserdem kann es bei dem oben beschriebenen Inhalator vorkommen, dass durch Feuchtigkeit, die in den Inhalator und in dessen Vorratskammer eindringt (z.B. durch versehentliches Husten des Benutzers in den Inhalator hinein), die ohnehin meist schlechte Fliessfähigkeit des Pulvers zusätzlich beeinträchtigt wird bzw. es kann zu "Klümpchenbildung" kommen.

Es ist daher die Aufgabe der Erfindung, einen Inhalator für Festkörper zu entwerfen, der die obenerwähnten Nachteile vermeidet, der eine schnelle, einfache und zuverlässige Dosierung der Festkörper ermöglicht, der eine Lagerung der Festkörper in einem gesonderten Vorratsbehälter, z.B. einer Kapsel, ermöglicht, und der einfach bedienbar ist. Ausserdem soll der Inhalator für eine grosse Anzahl von Inhalationen ausgelegt sein, also nicht als sogenannter Wegwerf-Inhalator ausgebildet sein.

Diese Aufgabe wird durch die Erfindung, wie sie im kennzeichnenden Teil von Patentanspruch 1 definiert ist, gelöst. Der Dosierstift ragt durch eine Oeffnung der Vorratskammer hindurch in diese hinein. Vorratskammer und Dosierstift sind zueinander relativ so bewegbar, dass sich die in den Dosierstift eingelassene kerbe in einer ersten Relativposition in der Vorratskammer befindet und in einer zweiten Relativposition sich im Luftkanal befindet. Dadurch wird erreicht, dass der Dosierstift in der ersten Relativposition in den Festkörpervorrat eingetaucht ist. Für die Festkörper wird dabei im folgenden beispielhaft ein nicht oder nur schlecht liessfähiges Pulver betrachtet, das beim Befüllen der Dosierkerbe in der ersten Relativposition in diese kerbe hineingedrückt wird. Ausserdem kann das Pulver auch in einem gesonderten Vorratsbehälter in der Vorratskammer gelagert werden, z.B. in einer Kapsel. Das kann, wie bereits erläutert, für solche Pulver wichtig sein, deren pharmazeutische Wirkung bei einer Lagerung im ständigen Kontakt mit der Luft nachlässt. Ausserdem bietet die Lagerung des Pulvers in einer Kapsel auch Schutz gegen eventuell eindringende Feuchtigkeit, so dass eine "Klümpchenbildung" und somit eine weitere Beeinträchtigung der Fliessfähigkeit sowie eine Beeinträchtigung der pharmazeutischen Wirkung durch den Kontakt des Pulvers mit der Feuchtigkeit vermieden wird.

In einem Ausführungsbeispiel des erfindungsgemässen Inhalators ist der Dosierstift in Bezug auf den Gerätekörper des Inhalators fest angeordnet und die Vorratskaminer ist gegen den Dosierstift bewegbar angeordnet. Dieses Ausführungsbeispiel zeichnet sich durch eine einfache Konstruktion und eine einfache Handhabung aus, wie anhand der detaillierten Beschreibung noch genauer erläutert wird.

Eine weitere Ausgestaltung des erfindungsgemässen Inhalators umfasst ein separates Betätigungsorgan zum Bewegen der Vorratskammer. Dieses Betätigungsorgan bewegt beim Betätigen die Vorratskammer gegen die Kraft einer Rückstellfeder die Vorratskammer in die erste Relativposition zum Dosierstift. Die Wand der Vorratskammer ist mit einem Wandfortsatz versehen, der in dieser ersten Relativposition den Luftkanal verschliesst. In diesem Wandfortsatz ist ein Oeffnung vorgesehen, die nach dem Rückstellen der Vorratskammer in die zweite Relativposition den Luftkanal wieder freigibt. Durch diese Ausgestaltung des Inhalators wird erreicht, dass beim Befüllen der Dosierkerbe nicht inhaliert und auch nicht in den Luftkanal hineingeblasen (z.B. gehustet) werden kann, wodurch in dieser Relaivposition von Dosierstift und Vorratskammer ein Eindringen von Feuchtigkeit in den Luftkanal vermieden wird. Nach erfolgter Entnahme der Pulvermenge aus der Vorratskammer bzw. aus dem darin eingelagerten Vorratsbehälter wird die Vorratskammer in die zweite Relativposition zurückgestellt und damit gelangt die befüllte Dosierkerbe des Dosierstifts in den Luftkanal. Die Oeffnung im Wandfortsatz der Vorratskammer gibt den Luftkanal frei und die Inhalation kann durchgeführt werden.

Bei einer weiteren Ausgestaltung des erfindungsgemässen Inhalators ist das Betätigungsorgan noch mit einem Erschütterungselement versehen, das beispielsweise als Knackfeder ausgebildet sein kann. Dieses Erschütterungselement bewirkt, dass beim Erreichen der ersten Realtivposition von Dosierstift und Vorratskammer, also beim Befüllen der Dosierkerbe, die Vorratskammer erschüttert wird, und so auch für den Fall von ganz besonders schlecht fliessfähigen Pulvern die Dosierkerbe vollständig, sicher und schnell befüllt wird.

Um dem Benutzer offenkundig zu machen, dass er gerade eine Dosierung vorgenommen hat und auch um bei schlecht liessfähigen Pulvern die Erschütterung der Vorratskammer zu ermöglichen, während der Dosierstift mit der Dosierkerbe in das Pulver hineinragt, ist eine Weiterbildung des erfindungsgemässen Inhalators mit einer Arretierungseinrichtung versehen. Diese Arretierungseinrichtung hält die Vorratskammer bzw. den Dosierstift in der ersten Relativposition zueinander fest. Nach Beendigung der Dosierung kann die Arretierung durch Betätigung eines Entriegelungselements wieder gelöst werden. Auf diese Weise werden ausserdem unbeabsichtigte Mehrfachdosierungen vermieden.

In einer vorteilhaften Ausgestaltung der Erfindung weist der Inhalator einen gesonderten Saugansatz auf, in welchem sich der Querschnitt des Luftkanals zum Auslass hin vergrössert. Ein solcher Saugansatz wirkt umgekehrt wie eine Düse und bewirkt eine vollständige Desagglomeration des Pulvers, so dass dieses im Luftstrom bis in die Lunge des Benutzers gelangen kann. Auf diesen Saugansatz ist eine Verschlusskappe aufsetzbar, in der ein Trockenmittel vorgesehen ist, um eventuell eingedrungene Feuchtigkeit zu eliminieren.

Um zu verhindern, dass der Benutzer nach erfolgter Festlegung der Pulvermenge und nach dem Einbringen des Pulvers in den Luftkanal durch Hineinblasen (z.B. durch Husten) in den Inhalator das Pulver durch den Lufteinlass hinausbläst, ist in einer weiteren Ausgestaltung der Erfindung am Lufteinlass ein Rückschlagventil vorgesehen.

Eine weitere Ausgestaltung des erfindungsgemässen Inhalators ist mit einem rücksetzbaren Zähler versehen, der beim Erreichen der ersten Relativposition von Vorratskammer und Dosierstift inkrementiert wird. Der Benutzer kann so erkennen, wieviele Dosierungen er bereits vorgenommen hat beziehungsweise wieviele Dosierungen ihm noch verbleiben, und kann gegebenenfalls die Vorratskammer wieder auffüllen.

Besonders geeignet ist der Inhalator zur Inhalation von antiasthmatisch wirksamen Stoffen oder Stoffgemischen, beispielsweise für eine Mischung aus Formoterol und Lactose, wie weiter unten noch näher erläutert wird.

Eine Ausgestaltung der Erfindung von ganz erheblicher Bedeutung sieht die Axialverstellung des Dosierstiftes durch eine Relativverdrehung zwischen dem den Stift tragenden Gerätekörper oder Gehäuseteil einerseits und dem die Vorratskammer enthaltenden Gehäuseteil andererseits vor, wobei diese Drehbewegung über Schrägflächen zwangsweise in eine Axialbewegung umgesetzt wird. Der erfindungsgemässe Inhalator ist dazu dadurch gekennzeichnet, dass ein die Vorratskammer aufweisender Gehäuseteil und der den Dosierstift tragende Gerätekörper dadurch aus einer ersten zusammengeschobenen Position in eine zweite auseinandergezogene Position relativ zueinander verschiebbar sind, dass sie relativ zueinander verdrehbar sind und wenigstens eine Schrägfläche an dem Gerätekörper und/oder dem Gehäuseteil und ein daran geführtes Gegenstück die Drehbewegung in die Axialbewegung umsetzt. Dies führt zu einer wesentlich bequemeren Dosierbewegung, weil eine Drehbewegung sicherer durchführbar ist als eine relativ kurze Axialbewegung, bei welcher zwei Teile auseinandergezogen werden müssen.

Der Gehäuseteil oder Gerätekörper kann mehrere an seinem Umfang verteilt angeordnete Schräglächen gleicher Steigung und Länge und der relativ dazu verdrehbare Körper oder Teil die entsprechende Anzahl von damit zusammenwirkenden Vorsprüngen oder Gegenstücken aufweisen. Somit wird die Drehbewegung an mehreren Stellen gleichzeitig in die Axialbewegung umgesetzt, so dass ein symmetrischer Kraftangriff und eine präzise Axialverstellung während des Verdrehens erfolgt.

Es hat sich als günstig erwiesen, wenn jeweils vier sich über nahezu 90 Grad ersaeckende, auf gleicher Höhe liegende und in gleicher Richtung ansteigende Schrägflächen vorgesehen sind, die mit als Vorsprüngen ausgebildeten Gegenelementen zusammenwirken, und wenn am Ende der Schrägflächen in vertikaler Richtung orientierte Durchgänge, Nuten oder dergleichen für die Vorsprünge vorgesehen sind, so dass nach einer Verdrehung und dem Entlanggleiten der Vorsprünge an den Schrägflächen mit der daraus resultierenden Axialverstellung des Dosierstiftes der Gehäuseteil mit Vorratskammer und das Gegenstück in axialer Richtung zusammenschiebbar und in Ausgangslage zurückverstellbar sind. Die als Führungen dienenden Schräglächen verlaufen also auf einem Teil einer Schraubenlinie, enden aber an einem axialen Durchgang, so dass bei einer Verdrehung zunächst die gewünschte Axialverstellung mit dem Transport des dosierten Materiales aus der Vorratskammer in den Luftweg erfolgt, während für die Rückverstellung des Dosierstiftes in die Vorratskammer ein einfaches axiales Zusammenschieben des Gerätekörpers und des Gehäuseteiles genügen, weil am Ende der Drehbewegung keine eine solche Verstellung verhindernden Schrägflächen mehr vorhanden sind.

Damit einerseits die vorerwähnte axiale Zurückverstellung möglich ist, andererseits aber schon beim Beginn der Drehbewegung der Vorsprung, beispielsweise ein Nocken, zwangsweise über eine Schrägfläche geführt wird, kann im Verlauf der axialen Rückführnuten wenigstens ein sägezahnartig ausgebildeter Vorsprung (Zahn) angeordnet sein, dessen steil abfallende Seite in Flucht mit der wirksamen Schrägfäche für den Nocken angeordnet ist, und der Nocken kann über die ansteigende Schrägseite dieses Zahns in axialer Richtung verschiebbar und in Ausgangslage der nächsten Dosierbewegung hinter der steil abfallenden Seite angeordnet sein. Bei der axialen Verschiebung in Ausgangsstellung kann also der jeweilige Vorsprung (Nocken) über den sägezahnartig ausgebildeten Vorsprung (Zahn) innerhalb der axialen Rückführnut hinweggleiten, wird dann aber von der anderen Seite an einer entsprechenden entgegengesetzten Bewegung gehindert, so dass auch dieser sägezahnartige Vorsprung schon zu der Führungs- oder Schrägfläche gehört, die dazu beiträgt, die Drehbewegung der beiden Gehäuseteile relativ zueinander in eine Axialbewegung umzusetzen.

Die Schräglächen entsprechen zweckmässigerweise Bruchteilen eines Gewindeganges und sind insbesondere an der Innenseite eines Wandstückes des Gehäuseteiles angeordnet. In diesem Falle hat dann der Gerätekörper die mit den Schrägflächen zusammenwirkenden Vorsprünge. Ebenso können diese Schrägfächen natürlich an einem Wandstück des Gerätekörpers vorgesehen sein und das Gehäuseteil weist die mit den Schräglächen zusammenwirkenden Vorsprünge auf.

Die Ausführungsform des Inhalators, bei welcher das Einbringen der Festkörper durch Umsetzen einer Drehbewegung in eine axiale Verstellbewegung bewirkt wird, gestattet eine Ausgestaltung von erheblicher Bedeutung, bei der einerseits ein Verdrehen der Gehäuseteile entgegen der Dosierbewegung gesperrt wird, und bei der diese Drehbewegung gleichzeitig dazu ausgenutzt werden kann, den Inhalator derart zu erschüttern, dass die pulverförmigen Festkörper gut in die Dosierkerbe liessen können. Eine solche zweckmässige Weiterbildung kann darin bestehen, dass an Berührstellen zwischen den relativ zueinander verdrehbaren Gehäuseteilen ein Gesperre, eine Ratsche oder dergleichen angeordnet ist, die ein gegensinniges Verdrehen der Gehäuseteile entgegen der Dosierbewegung sperrt. Einerseits werden dadurch Fehlbedienungen ausgeschlossen, weil die Verdrehung für den Benutzer nur in einer Richtung möglich ist, und andererseits wird durch die Ratsche während des Verdrehens das Gerät genügend erschüttert, um ein gutes Fliessen des Pulvers in die Dosierkerbe zu ermöglichen.

Dabei kann das Gesperre ein Gesperrerad mit Sägezähnen an dem einen Gehäuseteil und mit jeweils in Umfangsrichtung gegeneinander versetzten Gegenzähnen oder Gruppen von Gegenzähnen an dem anderen Gehäuseteil oder Gerätekörper aufweisen, wobei der Versatz der jeweils vorgesehenen Gegenzähne in Umfangsrichtung gegeneinander derart gewählt ist dass die Rastschritte des Gesperres kleiner als die Zahnteilung ist. Es genügt bei einem solchen Gesperre, wenn jeweils ein Zahn des Gesperrerades mit einem Gegenzahn im Eingriff ist. Da an dem Inhalator mehrere in Umfangsrichtung gegeneinander versetzte Gegenzähne vorgesehen sind, deren Versatz von der Zahnteilung abweicht, ergeben sich entsprechend kleine Schritte in Drehrichtung, nach denen jeweils wieder eine Verrastung erfolgt. Entsprechend viele Erschütterungen treten auch während der gesamten Drehbewegung auf.

Dabei ist vorteilhaft, dass sich die Zähne, die Zahnlücken und die Gegenzähne geradlinig in axialer Richtung erstrecken. Sie behindern somit nicht die am Ende der Dosierbewegung durchzuführende Rückverstellung.

Um auf engem Raum einerseits die Führungs- oder Schrägflächen und andererseits die Ratsche oder das Gesperre unterbringen zu können, kann der die Dosiernadel aufweisende Gerätekörper oder Gehäuseteil bereichsweise eine Doppelwandung haben, deren innere Wandung die mit den Schräglächen an dem anderen Gehäuseteil zusammenwirkenden Vorsprünge trägt, die von dieser inneren Wandung radial nach aussen abstehen und die äussere Wandung kann die die Schrägflächen aufweisenden Wandteile des anderen Gehäuseteiles aussen umgreifen und die Gegenzähne für die an der Aussenseite des die Vorratskammer enthaltenden Gehäuseteiles angeordneten Gesperrerades beaufschlagenden Gegenzähne enthalten. Der Gerätekörper hat also praktisch zwei Wandungen, in deren Zwischenraum eine entsprechende Wandung des Gehäuseteiles eingreifen kann, die dann auf der einen Seite die Schräglächen und auf der anderen, aussenliegenden Seite ein Teil des Gesperres tragen kann. Eine solche sehr platzsparende Anordnung unterschiedlicher zusammenwirkender Komponenten des gesamten Systemes ist insbesondere dadurch möglich, dass die Teile aufgrund ihrer relativen Verdrehbarkeit gegeneinander kreisrund sind, also eine konzentrische Anordnung der unterschiedlichen Wandungen erlauben.

Während die innere Wandung des die Dosiernadel aufweisenden Gehäuseteiles und die die Führungsflächen aufweisenden Wand des die Vorratskammer aufweisenden Gehäuseteiles - zumindest im Bereich ihres Zusammenwirkens - kreisrund sind, kann die äussere Wandung des ersteren Gehäuseteiles einen von dieser Kreisform abweichenden Querschnitt haben, so dass Eckbereiche zwischen den Wandungen gebildet sind, und insbesondere bei verdrehten und/oder axial auseinandergeschobenen Gehäuseteilen kann die Luft über diese dann offenen Eckbereiche angesaugt und über Durchbrüche an den inneren Wandteilen sowie über wenigstens eine Rückschlagkappe zu den Dosierkanälen und der Dosiernadel geführt sein. Diese von einer Kreisform abweichende Querschnittsform der äusseren Wandung erlaubt auch, an ihrer Innenseite jeweils Gegen-Rastzähne vorzusehen, die so gegeneinander in Umfangsrichtung versetzt sind, dass das Gesperrerad schon nach weniger als einer Zahnteilung an einem Gegenzahn ein Widerlager findet.

Ferner gelangen diese Eckbereiche beim Verdrehen ausserhalb des Gesamtumrisses, so dass der Luftzutritt erleichtert wird.

Die Stirnseite der Aussenwandung des die Dosiernadel enthaltenden Gehäuseteilses kann in zusammengeschobener Position gegen eine Anschlagfläche des anderen Gehäuseteiles mit übereinstimmender umlaufender Kontur anliegen und in dieser Position kann der Luftweg abgeschnitten und das Innere des Inhalators luftdicht verschlossen sein. Dies hat den Vorteil, dass keine Feuchtigkeit zutreten kann, so lange das Gerät unbenutzt ist, so dass Anbackungen des zu dosierenden Pulvers bei der nächsten Benutzung aufgrund von Feuchtigkeit vermieden werden.

Die erfindungsgemässe Verdrehbarkeit der beiden Gehäuseteile relativ zueinander zum Durchführen der Axialverstellung des Dosierstiftes erlaubt eine weitere vorteilhafte und zweckmässige Ausgestaltung dahingehend, dass der eine Gehäuseteil einen relativ zu einem Anzeigefenster verdrehbaren Anzeigering enthalten kann, der durch die Relativ-Verdrehung der beiden Gehäuseteile zueinander beim Dosieren jeweils um einen kleinen Winkelbetrag gleicher Grösse und gleicher Richtung weiterbewegbar ist. Somit kann der Benutzer indirekt über diesen Anzeigering den Entleerungsfortschritt feststellen, also rechtzeitig einen neuen Inhalator in Benutzung nehmen oder den vorhandenen Inhalator - sofern möglich - nachfüllen.

Der Anzeigering kann als Zahnrad ausgebildet sein, welches exzentrisch zu einem Hohl-Zahnrad oder einer Hohlverzahnung in dem die Dosiernadel aufweisenden Gehäuseteil gelagert ist und mit seiner Aussenverzahnung in die Innenverzahnung dieses Hohlrades passt, wobei die Zähnezahl dieses inneren Zahnrades gegenüber der Zähnezahl des Hohlrades so wenig abweicht, dass das Innenzahnrad gegenüber dem Hohl-Zahnrad um etwa eine einzige Umdrehung oder einen Bruchteil davon verdrehbar ist, wenn eine vorbestimmte Anzahl von zum Beispiel 200 Dosierungen durchgeführt ist. Die zahlreichen Teil-Drehbewegungen beim jeweiligen Dosieren führen also zu einem sehr langsamen allmählichen Verdrehen des Anzeigeringes, der durch bestimmte Farbgebungen oder dergleichen dem Benutzer an dem Anzeigefenster die Abnahme des zu inhalierenden Feststoffes signalisieren kann. Dabei ist wiederum vorteilhaft, dass an dem Gerät Drehbewegungen immer nur in einer Richtung stattfinden und Rückverdrehungen gesperrt sind.

Um zu erreichen, dass der Benutzer nicht weiter zu inhalieren versucht, wenn die vorgesehene Höchstmenge an Einzeldosierungen stattgefunden hat, kann das als Anzeigering dienende Zahnrad und damit die Dosierbewegung nach Erreichen der vorgesehenen Anzahl von Dosierungen blockierbar sein. Dies lässt sich auf unterschiedliche Weise erreichen.

Eine zweckmässige Möglichkeit, die gleichzeitig die durch das Verdrehen auch bewirkte Axialbewegung mit ausnutzt, kann darin bestehen, dass das als Anzeigering dienende Zahnrad einen Anschlag aufweist, der mit einem Gegenanschlag des relativ zu ihm verdrehbaren Gehäuseteiles in dem Sinne zusammenwirkt, dass die beiden Anschläge in Ausgangslage einander nahe sind oder sich berühren und durch die Dosierbewegungen allmählich in Umfangsrichtung auseinanderbewegbar sind, wobei durch die gleichzeitige Axialverstellung diese beiden Anschläge jeweils in zwei gegeneinander höhenversetzten Ebenen zu liegen kommen und durch die Rückbewegung wieder in eine übereinstimmende Ebene gelangen, und dass die beiden - zweckmässigerweise als Vorsprünge gestalteten - Anschläge nach etwa einer Umdrehung des Anzeigeringes und einem letzten Dosiervorgang in den beiden Ebenen derart übereinanderliegen, dass die axiale Rückverstellung in Ausgangslage und damit ein erneuter Dosiervorgang gesperrt ist.

Eine weitere Ausgestaltung der Erfindung von erheblichem Vorteil kann darin bestehen, dass in der Vorratskammer auf der dem Dosierstift entgegengesetzten Seite ein von einer schwachen Feder beaufschlagter, der Abnahme des Kammerinhaltes folgender Kolben oder dergleichen angeordnet ist, wobei die Federkraft so gering ist, dass eine Kompression der Festkörper verhindert ist, wobei die Federkraft aber so stark ist, dass sie den Kolben auch gegen die Schwerkraft der Festkörper und seines eigenen Gewichtes der Abnahme des Kammervolumens folgend verschiebt. Durch diese Anordnung ist es möglich, den Inhalator in praktisch belie-

EP 0 505 321 A2

biger Lage zu benutzen und dennoch bei jeder Dosierbewegung auch ein Füllen der Dosierkerbe sicherzustellen. Darüber hinaus wird durch diese Massnahme und das ständige leichte Nachschieben des pulverförmigen Festkörpers dessen Zusammenbackung oder eine Kanalbildung an der Stelle, an der der Dosierstift ein- und austritt, verhindert. Die einen Nachführkolben beaufschlagende Feder bewirkt somit, dass bei jeder Dosierbewegung, am besten nach einem vorangehenden Schütteln des Inhalators, auch wirklich eine vollständige Dosierung erfolgt.

Die Vorratskammer kann innerhalb des Gehäuseteiles lösbar und auswechselbar angeordnet sein und/oder einen lösbaren Deckel haben. Somit kann nach dem Entleeren der Vorratskammer entweder diese Vorratskammer selbst ersetzt werden oder sie kann nachgefüllt werden, statt dass ein vollständig neuer Inhalator benutzt und der entleerte weggeworfen werden muss.

Die auswechselbar in dem Gehäuseteil angeordnete Vorratskammer kann in Ausgangslage vor der Montage einen durchstossbaren und/oder über Sollbruchstellen dicht befestigten Verschluss des Durchtrittskanales für den Dosierstift haben, welcher Verschluss durch das Einfügen der Vorratskammer in ihr Gehäuseteil mittels des Dosierstiftes automatisch öffenbar ist. Wird also eine entleerte Vorratskammer durch eine solche neue Vorratskammer ersetzt, bewirkt ihr Einfügen in den Gehäuseteil sogleich auch die Einbringung des Dosierstiftes in diese Vorratskammer, ohne dass diese zuvor schon offen sein muss.

Damit der durch den Dosierstift öffenbare Verschluss beim späteren Gebrauch nicht in die Dosierkerbe gelangen und die Dosierbewegungen stören kann, ist es zweckmässig, wenn der durch diese Montage öffenbare Verschluss der Vorratskammer in das Innere der Vorratskammer aufschwenkbar ist und sein Durchmesser grösser als die axiale Ersaeckung der Dosierkerbe ist. Hier sind allerdings auch andere technische Ausgestaltungen dieses Prinzips möglich.

Nachstehend sind Ausführungsbeispiele der Erfindung anhand der Zeichnung näher erläutert. Es zeigt in zum Teil schematisierter Darstellung:

Fig. 1 einen Längsschnitt eines erfindungsgemässen Inhalators im Ausgangszustand (zweite Relativposition),

Fig. 2 den Längsschnitt durch den Inhalator aus Fig. 1 in der ersten Relativposition,

Fig. 3 eine Frontansicht eines Oberteils des Inhalators (in die Zeichenebene der Fig. 2 hinein),

Fig. 4 eine Seitenansicht des Oberteils gemäss dem Pfeil IV der Fig. 3,

Fig. 5 eine Aufsicht auf ein Unterteil des Inhalators gemäss dem Pfeil V der Fig. 2,

Fig. 6 einen Längsschnitt durch eine Vorratskammer des Inhalators (Schnittebene identisch mit der Schnittebene der Fig. 1 und 2),

Fig. 7 einen Schnitt durch die Vorratskammer längs der Linie VII-VII der Fig. 6,

Fig. 8 einen weiteren Schnitt durch die Vorratskammer längs der Linie VIII-VIII der Fig. 6,

Fig. 9 die äussere Gestalt des Inhalators,

Fig. 10 einen Schnitt längs der Linie X-X der Fig. 11 durch die Arretierungseinrichtung im Ausgangszustand (zweite Relativposition)

Fig. 11 eine Aufsicht auf die Arretierungseinrichtung gemäss dem Pfeil XI in Fig. 10

Fig. 12 einen Schnitt längs der Linie XII-XII der Fig. 13 durch die Arretierungseinrichtung in der ersten Relativposition,

Fig. 13 eine Aufsicht auf die Arretierungseinrichtung gemäss dem Pfeil XIII in Fig. 12,

Fig. 14 einen Längsschnitt eines abgewandelten erfindungsgemässen Inhalators in der ersten Relativposition, in welcher die Dosierkerbe in der Vorratskammer positioniert ist, wobei die Axialverstellung über eine Verdrehung des den Dosierstift tragenden Gerätekörpers gegenüber dem die Vorratskammer enthaltenden Gehäuseteil durchführbar ist,

Fig.15 einen teilweise schematisierten Querschnitt des Inhalators gemäss der Linie D-D in Fig. 14,

Fig.16 eine abgewickelte Ansicht einer über beispielsweise etwa einen Viertel-Umfang reichenden Schrägfläche an dem Gehäuseteil, über welche mit Hilfe eines Vorsprungs an dem Gerätekörper die Drehbewegung in die erforderliche Axialverstellung des Dosierstiftes aus seiner in Fig. 14 dargestellten Ausgangslage in die in Fig. 19 dargestellten Position umgesetzt wird,

Fig. 17 einen Schnitt gemäss der Linie C-C in Fig. 15 durch eine innerhalb des Luftweges liegende Rückschlagklappe,

Fig. 18 einen Teil-Schnitt gemäss der Linie B-B in Fig. 15 durch einen zu dem Dosierstift führenden Kanal mit Ansicht auf die Eintritte in zwei weitere Kanäle,

Fig. 19 einen Längsschnitt des Inhalators gemäss Fig. 14 nach Durchführung einer in eine Axialverstellung umgesetzten Drehbewegung,

Fig.20 einen Querschnitt gemäss der Linie E-E in Fig. 19 durch ein zwischen den relativ zueinander verdrehbaren Gehäuseteilen vorgesehenes Gesperre,

Fig.21 einen der Fig. 19 entsprechenden Längsschnitt des Inhalators, wobei der rechte Teil gegenüber der

6

Anordnung in den Figuren 14 bis 19 verdreht gezeichnet ist, so dass dort der Schnitt durch die Rückschlagkappe und die Luftkanäle verläuft,

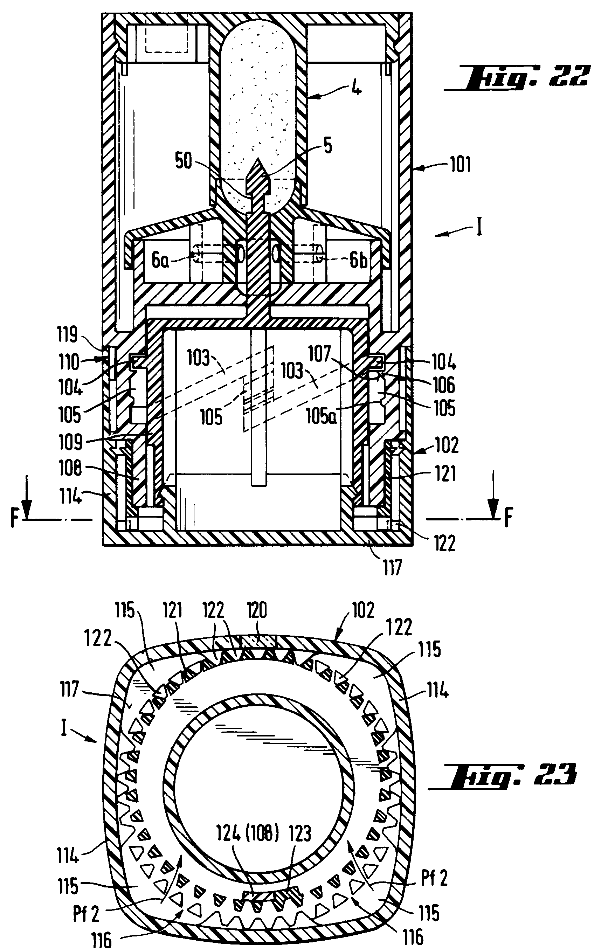

Fig.22 einen gegenüber Fig. 14 um 90 Grad verdrehten Längsschnitt des Inhalators in der ersten Relativposition,

Fig.23 einen Querschnitt gemäss der Linie F-F in Fig.22 durch eine Anzeigevorrichtung,

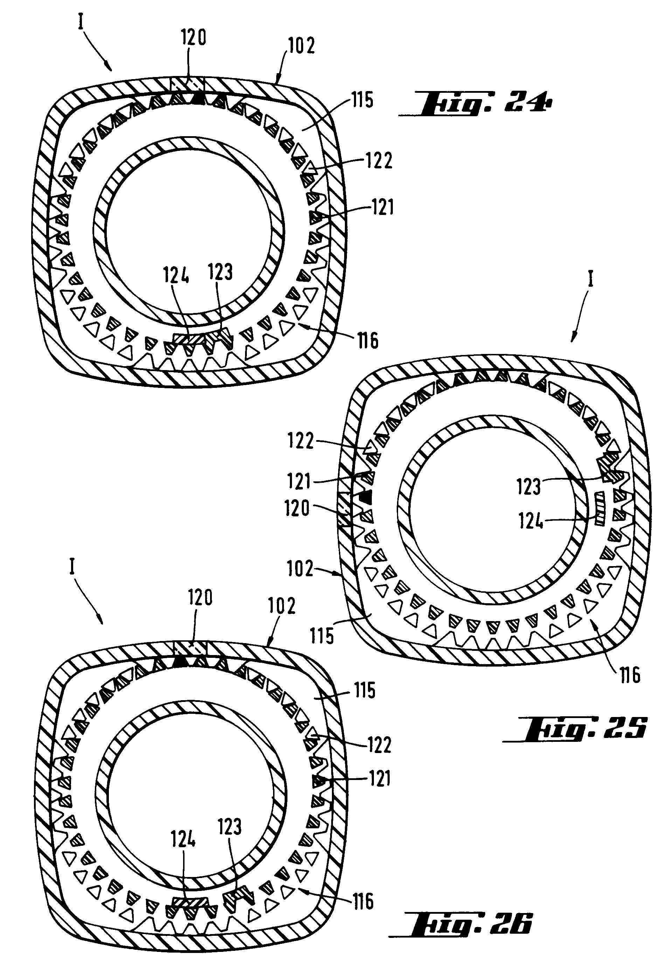

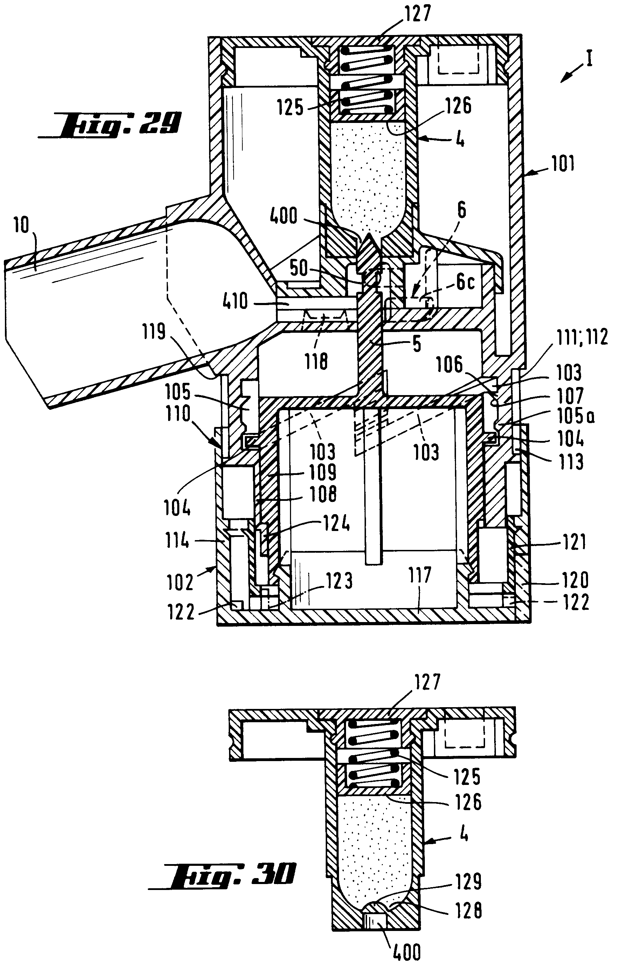

Fig.24 bis Fig.28 die Anzeigevorrichtung gemäss Fig.23 in unterschiedlichen Positionen jeweils nach einer zunehmenden Anzahl von Dosierungen, wobei in Fig.28 eine Blockierung nach der vorgesehenen Höchstzahl von Dosierungen dargestellt ist,

Fig.29 einen Längsschnitt eines abgewandelten Inhalators in der zweiten Relativposition, wobei in der Vorratskammer ein federbelasteter Nachführ-Kolben für die in der Vorratskammer befindlichen Festkörper vorgesehen ist, sowie

Fig.30 einen Längsschnitt durch eine auswechselbare Vorratskammer mit einem über Sollbruchstellen oder dergleichen geschlossenen, durch das Einfügen in den Inhalator mittels des Dosierstiftes automatisch öffenbaren Verschluss, wobei in diesem Ausführungsbeispiel ebenfalls ein federbelasteter Nachführ-Kolben vorgesehen ist.

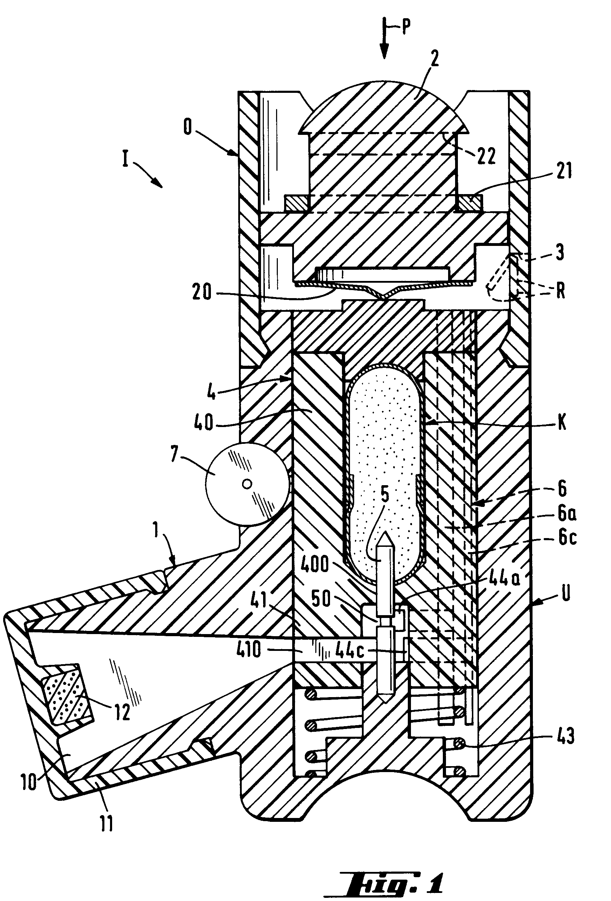

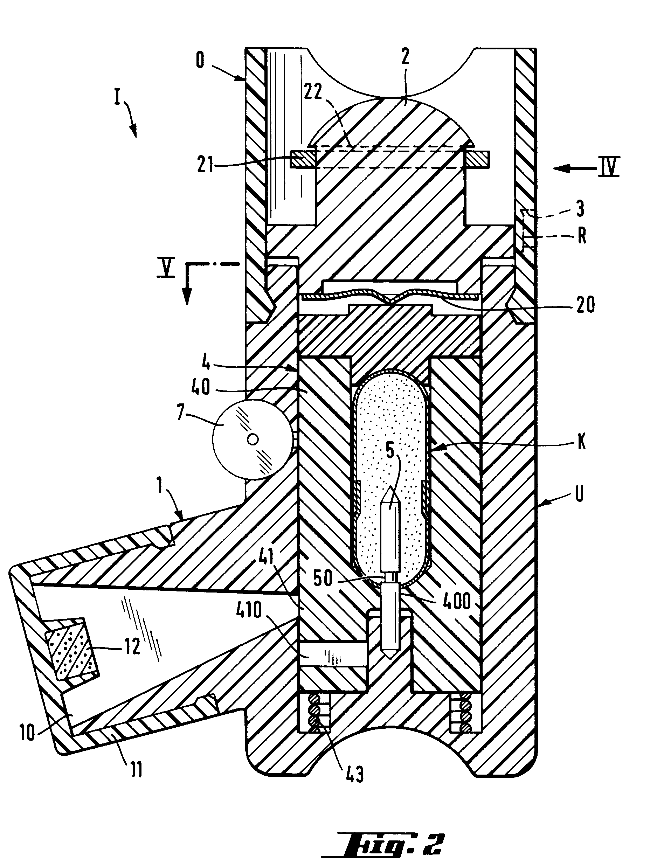

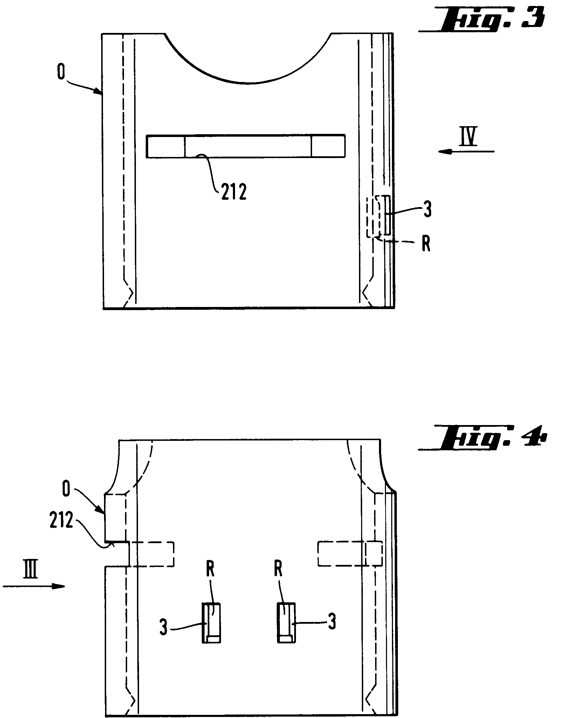

Anhand der in den Fig. 1 und 2 gezeigten Längschnitte durch den Inhalator soll dessen Aufbau und Funktionsweise genauer erläutert werden. Der Inhalator I umfasst im wesentlichen einen Lufteinlass 3 mit einem angedeuteten Rückschlagventil R (beides strichliert dargestellt, da nicht in der Schnittebene liegend) in seinem abnehmbaren Oberteil O, ein Betätigungsorgan in Form eines Druckknopfs 2, eine Vorratskammer 4 für die Festkörper oder für einen gesonderten Vorratsbehälter, zum Beispiel eine Kapsel K, in der die Festkörper eingelagert sind, einen Dosierstift 5, der mit einer hier beispielhaft nutförmig ausgebildeten Dosierkerbe 50 versehen ist, einen Luftkanal 6 (strichliert dargestellt, da nicht in der Schnittebene liegend), sowie einen Saugansatz 1, der mit einem Auslass 10 für das Festkörper-Luft-Gemisch versehen ist, welches in noch zu erläuternder Art und Weise im Inhalator erzeugt wird. Auf den Saugansatz 1 ist eine Schutzkappe 11 aufgesetzt, mit deren Hilfe der Auslass 10 verschliessbar ist.

Bei der nun folgenden Erläuterung der Funktionsweise des Inhalators wird der Fall betrachtet, dass die Festkörper in einer Kapsel K eingelagert sind, die beispielsweise 100 Dosierungen enthält, und dass diese Kapsel K bereits in die Vorratskammer 4 eingebracht ist. Die Abmessungen der Kapsel K und die Abmessungen der Vorratskammer 4 sind dabei aufeinander abgestimmt. Setzt man nun den Druckknopf 2 ein und das Oberteil O auf, und drückt man den Druckknopf 2 in seine Ausgangsstellung, so durchstösst der Dosierstift 5 durch die Oeffnung 400 der Vorratskammer 4 hindurch die Kapsel K und ragt in die in der Kapsel K eingelagerten Festkörper hinein. Der Inhalator ist nun in seinem Ausgangszustand (Fig. 1), von dem aus zunächst die Dosierung der Festkörpermenge und anschliessend die Inhalation erfolgen kann.

Die Vorratskammer 4 mit der eingelagerten Kapsel K befindet sich in der Ruhestellung, wie sie in Fig. 1 dargestellt ist. Vorratskammer 4 und Dosierstift 5 sind in dieser Ruhestellung relativ zueinander so angeordnet, dass der Dosierstift 5 zwar in das Innere der Kapsel K hineinragt und die Oeffnung 400 der Vorratskammer 4 verschliesst, die nutförmige Dosierkerbe 50 sich jedoch noch im Luftweg zwischen Einlass 3 und Auslass 10 befindet. Diese Relativposition (Ausgangszustand) von Dosierstift 5 und Vorratskammer 4 ist im folgenden als zweite Relativposition bezeichnet. Die Wand 40 der Vorratskammer 4 ist mit einem Wandfortsatz 41 versehen, in dem eine Oeffnung 410 vorgesehen ist. In dieser Relativposition von Vorratskammer 4 und Dosierstift 5 gibt diese Oeffnung 410 den Luftweg von Lufteinlass 3 und Auslass 10 über den Luftkanal 6 frei. Der Wandfortsatz 41 der Vorratskammer 4 stösst an seinem unteren Ende gegen eine Rückstellfeder 43, die in dieser zweiten Relativposition entspannt ist, sieht man von der notwendigen Vorspannung des Mechanismus ab.

Zum Durchführen einer Dosierung drückt der Benutzer nun gemäss dem Pfeil P von oben auf den Druckknopf 2. Dieser Druckknopf 2 ist in dem hier erläuterten Ausführungsbeispiel mit einem Erschütterungselement versehen, das als Knackfeder 20 ausgebildet ist. Die Knackfeder 20 ist am Druckknopf 2 befestigt, z.B. kann sie angeklebt sein. Auf die Wirkung dieser Knackfeder 20 wird zu einem späteren Zeitpunkt noch genauer eingegangen. Für den Augenblick ist nur von Bedeutung, dass die Federhärte dieser Knackfeder 20 grösser ist als die mittlere Federhärte der Rückstellfeder 43. Durch den Druck von oben auf den Druckknopf 2 gemäss dem Pfeil P wird die Vorratskammer 4 abwärts bewegt und die Rückstellfeder 43 wird zusammengedrückt. Die Knackfeder 20 bleibt aufgrund der grösseren Federhärte noch in ihrem Ruhezustand. Der Dosierstift 5, der bezüglich des Gerätekörpers ortsfest angeordnet ist (beispielsweise in eine Bohrung eingepasst ist), bohrt sich aufgrund der Abwärstbewegung der Vorratskammer 4 tiefer in die Festkörper hinein, die in der Kapsel K eingelagert sind. Wenn die Rückstellfeder 43 beinahe ganz zusammengedrückt ist, befindet sich die nutförmige Dosierkerbe 50 des Dosierstifts 5 bereits im Innern der Kapsel K und damit in den Festkörpern.

Schon während des Eindringens der Dosierkerbe 50 des Dosierstifts 5 in die Vorratskammer 4, insbesondere aber auch beim Erreichen der Position, in der die Rückstellfeder 43 ganz zusammengedrückt ist und die im folgenden als erste Relativposition bezeichnet ist, wird die nutförmige Dosierkerbe 50 des Dosierstifts 5 mit Festkörpern befüllt. Um zu erreichen, dass auch bei extrem schlecht fliessfähigen und sehr feinkörnigen Pulvern, die eingangs schon erwähnt worden sind, die Dosierkerbe 50 schnell, zuverlässig und vollständig befüllt wird, springt die knackfeder 20, durch die auf dem letzten Wegstück zunehmende Federhärte der fast ganz zusammengedrückten Rückstellfeder 43 bedingt, ruckartig in den in Fig. 2 dargestellten Zustand um. Durch dieses ruckartige Umspringen der knackfeder 20 wird die Vorratskammer 4 und mit ihr die eingelagerte Kapsel K und das Pulver erschüttert, so dass auch extrem schlecht liessfähige Pulver in die Dosierkerbe 50 hineingelangen. Alternativ könnte natürlich auf die knackfeder 20 verzichtet werden und der Benutzer könnte den Inhalator ruckartig schütteln, um den beschriebenen Effekt zu erzielen.

Befinden sich nun die Vorratskammer 4 und der Dosierstift 5 in der in Fig. 2 gezeigten ersten Relativposition zueinander, so verschliesst der Wandfortsatz 41 den Weg des Luftstroms. Dadurch wird erreicht, dass durch ein Hineinblasen in den Saugansatz 1, z.B. durch unbeabsichtigtes Husten, während der Dosierung keinerlei Pulver aus dem Inhalator durch den Lufteinlass 3 herausgeblasen werden kann. Ausserdem wird durch dieses Verschliessen des Luftwegs erreicht, dass während der Dosierung, also während der Befüllung der Dosierkerbe 50, auch keine Feuchtigkeit in den Inhalator gelangen kann, was durch unbeabsichtigtes Husten des Benutzers sonst durchaus vorstellbar ist.

Nach dem Umspringen der knackfeder 20 ist die Dosierkerbe 50 mit dem Pulver befüllt. Wird nun der Druckknopf 2 losgelassen, so bewegt die Rückstellfeder 43 die Vorratskammer 4 in die zweite Relativposition, also in die Ausgangsstellung, zurück. In dieser zweiten Relativposition (Ausgangsstellung) gibt die Oeffnung 410 im Wandfortsatz 41 der Vorratskammer 4 den Luftweg frei, wie bereits beschrieben. Saugt der Benutzer nun am Saugansatz 1 Luft an, so wird durch den Lufteinlass 3 und durch das in Fig. 1 symbolisch durch die klappe R strichliert angedeutete Rückschlagventil R hindurch im Luftkanal 6 ein Luftstrom erzeugt. Dieser Luftstrom bläst die dosierte Pulvermenge aus der Dosierkerbe 50 heraus. Auf diese Weise entsteht das Festkörper-Luft-Gemisch, welches dann durch den Auslass 10 am Saugansatz 1 vom Benutzer inhaliert wird und in dessen Atemwege gelangen kann. Um ein möglichst effektives Ausblasen der dosierten Pulvermenge aus der Dosierkerbe 50 zu erreichen und um die Geschwindigkeit des Festkörper-Luft-Gemischstroms beim Eintritt in die Atemwege des Benutzers zu verringern, vergrössert sich im Saugansatz 1 der Querschnitt des ansonsten klein gehaltenen Luftkanals zum Auslass 10 hin.

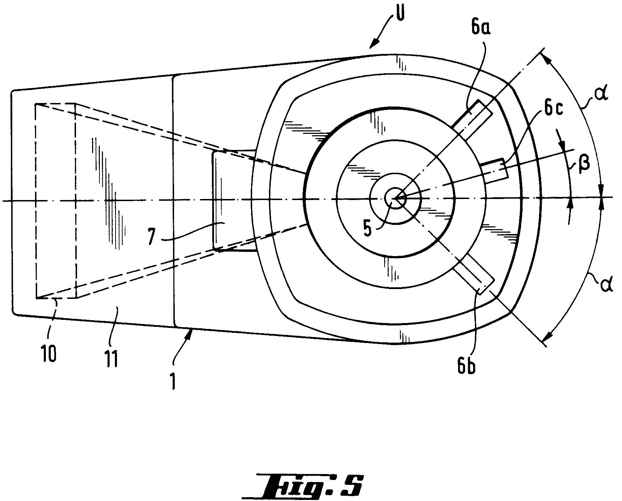

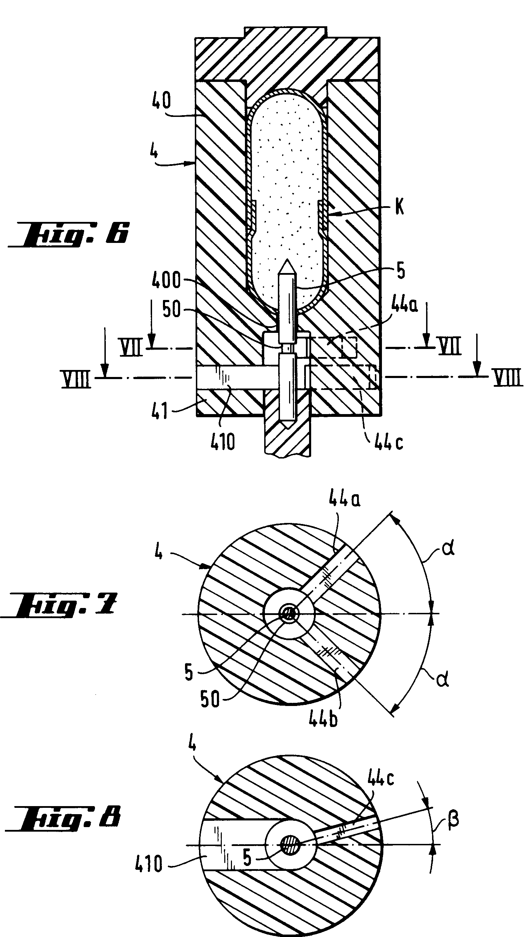

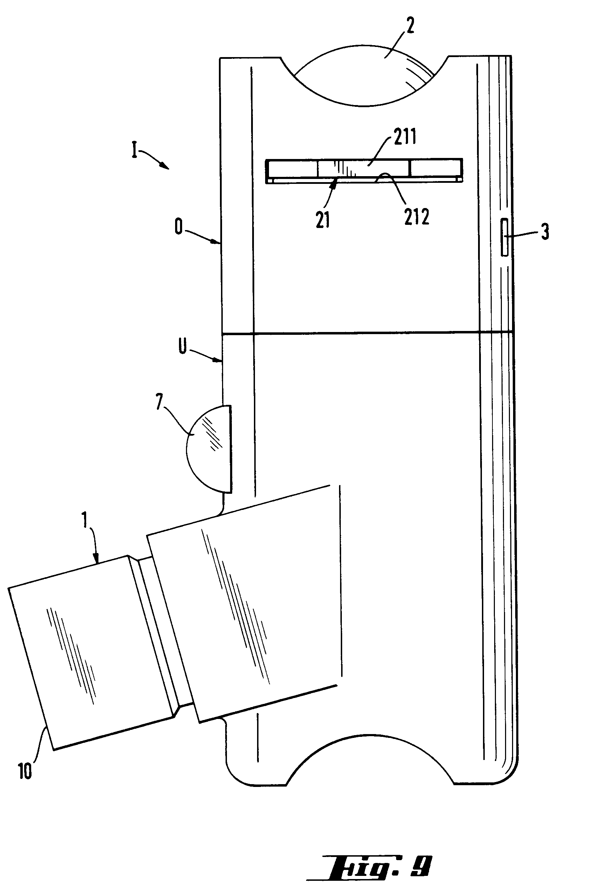

Wie der Luftkanal 6 im einzelnen in dem hier erläuterten Ausführungsbeispiel ausgebildet ist, bzw. wie der Luftstrom im Inhalator geführt ist, soll im folgenden anhand der Fig. 3 bis 9 genauer erläutert werden. Die Fig. 3 und 4 zeigen das Oberteil O des Inhalators, wobei in der Seitenansicht in Fig. 4 die Lufteinlässe 3 (längliche Schlitze) zu erkennen sind. Durch diese Lufteinlässe 3 und durch das Rückschlagventil R hindurch (in Fig. 1 sowohl in seiner Ruhestellung als auch in der Stellung beim Einatmen strichliert angedeutet) gelangt die angesaugte Luft in das Innere des Inhalators.

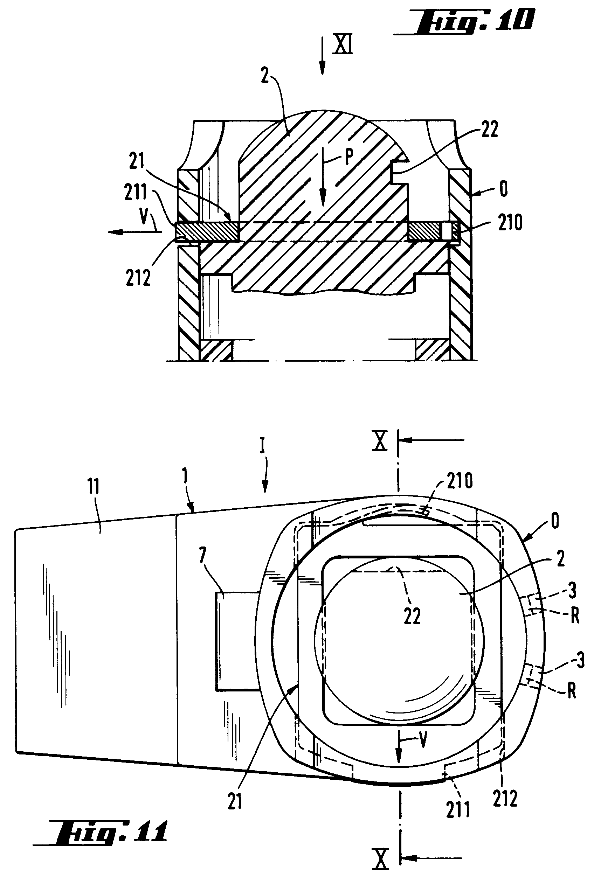

Wie der Luftstrom im Inneren des Inhalators weitergeführt ist, kann man aus Fig. 5 erkennen, die eine Aufsicht auf ein Unterteil U des Inhalators zeigt. Man erkennt drei eingelassene Luftkanäle 6a, 6b, 6c, die im Körper des Unterteils U des Inhalators abwärts führend eingelassen sind (siehe auch strichlierte Linie 6 in Fig. 1) und durch die der Luftstrom weitergeführt ist.

Fig. 6 zeigt die mittels des Druckknopfs 2 bewegbare Vorratkammer 4, hier allerdings ohne eine eingelegte Kapsel K. Nach der Beendigung eines Dosiervorgangs, wenn sich also Dosierstift 5 und Vorratskammer 4 in der Ausgangsstellung befinden (zweite Relativposition), wird mittels Luft das Pulver aus der Dosierkerbe 50 des Dosierstifts 5 herausgeblasen. Dazu muss der Luftstrom allerdings aus den Luftkanälen 6 zur Dosierkerbe 50 gelangen.

Wie dies realisiert sein kann, ist in den Fig. 7 und 8 dargestellt, die jeweils Schnitte längs den Linien VII-VII bzw. VIII-VIII zeigen. Die Schnittebene VII-VII verläuft dabei genau auf der Höhe der Dosierkerbe, so dass diese optimal im Luftstrom steht. Man erkennt anhand der Fig. 7, dass zwei Lufteintrittsöffnungen 44a und 44b für den Luftstrom unter dem gleichen Winkel $\alpha$ bezüglich der Schnittebene des Längsschnitts aus Fig. 1 angeordnet sind wie die entsprechenden zwei Luftkanäle 6a und 6b in Fig. 6.

Die dritte Lufteintrittsöffnung 44c ist unter dem gleichen Winkel $\beta$ bezüglich dieser Schnittebene angordnet wie der entsprechende Luftkanal 6c, allerdings wie in Fig. 8 zu erkennen ist, unterhalb der beiden anderen Lufteintrittsöffnungen 44a und 44b in der Schnittebene VIII-VIII. Diese Lufteintrittsöffnung 44c dient dazu, um eventuell aus der Dosierkerbe herausgefallene Festkörper- bzw. Pulverkörner mit auszublasen und so dem Luftstrom beizumischen. Durch die Oeffnung 410 im Wandfortsatz 41 der Vorratkammer 4 hindurch kann das gebildete Festkörper-Luft-Gemisch durch den Auslass 10 im Saugansatz 1 hindurch dann in die Atemwege des Benutzers gelangen.

Um unbeabsichtigte Mehrfachdosierungen zu vermeiden, wird die Vorratskammer 4 und der Dosierstift 5 (siehe Fig. 2) durch eine Arretierungseinrichtung in der ersten Relativposition zueinander festgehalten. Diese Arretierungseinrichtung ist in Fig. 1 beispielhaft durch einen Schieber 21 angedeutet, dessen Funktionsweise im folgenden anhand der Fig. 9 bis 13 erläutert wird.

Um die Funktionsweise der Arretierungseinrichtung verstehen zu können, ist zunächst in Fig. 9 noch einmal die äussere Gestalt des Inhalators dargestellt, wobei im Oberteil O, wie auch schon in der Fig. 3 zu erkennen ist, eine Oeffnung 212 vorgesehen ist.

In Fig. 10 ist der Druckknopf 2 in einer Seitenansicht im Längsschnitt dargestellt. Er ist unterhalb seines "Kopfs" mit einer Nut 22 versehen, die schon in Fig. 1 strichliert angedeutet ist. In diese Nut 22 ist ein Schieber 21, der ebenfalls in Fig. 1 dargestellt ist, in noch zu erläuternder Art und Weise einrastbar.

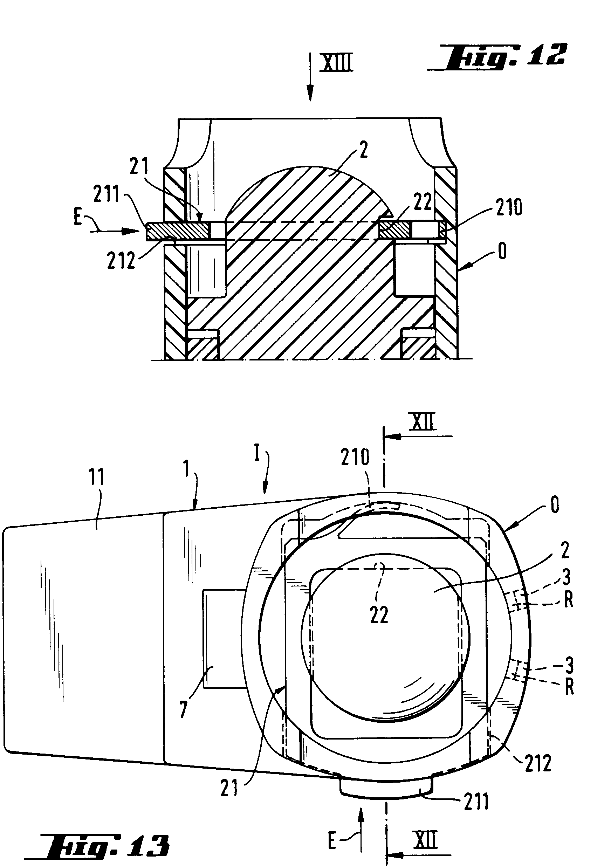

In seinem Ausgangszustand (zweite Relativposition von Vorratskammer 4 und Dosierstift 5) ist der Inhalator in Fig. 11 in einer Aufsicht dargestellt. In dieser Aufsicht ist erkennbar, dass der Schieber 21 mit einer Blattfeder 210 und einem Halteknopf 211 versehen ist. Die Blattfeder 210 stützt sich gegen eine Nut in der Innenwand des Inhalators ab (siehe auch Fig. 10) und versucht den Schieber 21 in Richtung des Pfeils V zu bewegen und den Halteknopf 211 noch weiter durch die in Fig. 9 und Fig. 10 bereits gezeigte Oeffnung 212 herauszudrücken.

Dieses weitere Herausdrücken des Halteknopfs 211 durch die Oeffnung 212 wird dadurch verhindert, dass der Schieber 21 in der Ausgangszustand gegen den Körper des Druckknopfs 2 (Betätigungsorgan) stösst. Dies ist besonders gut aus der Schnittdarstellung in Fig. 10 erkennbar.

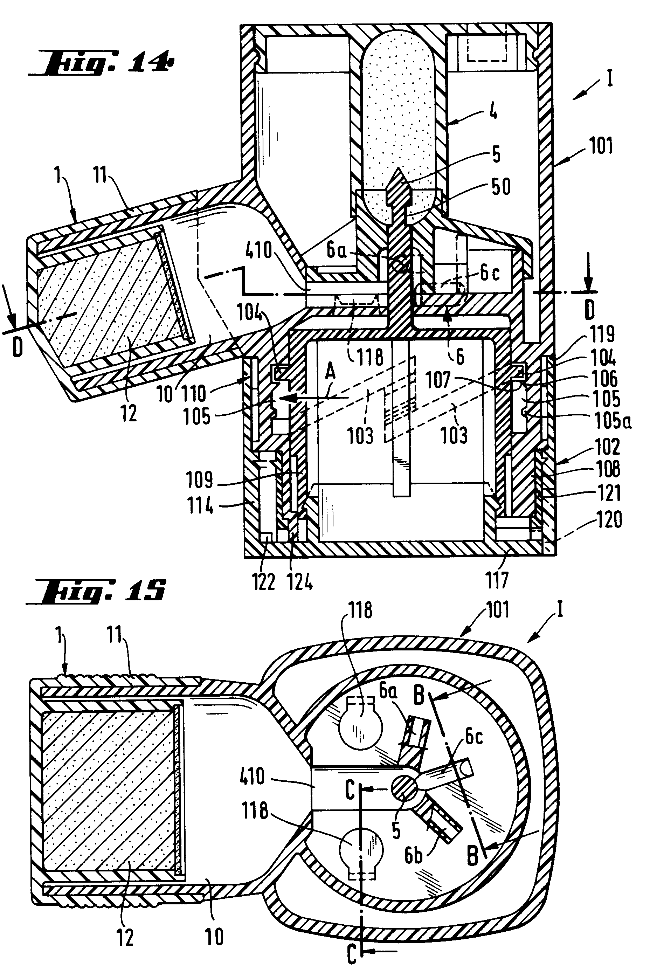

Wird nun der Druckknopf 2 betätigt (Pfeil P in Fig. 1), also die Dosierkerbe 50 des Dosierstifts 5 in die Vorratskammer 4 hineinbewegt, so bleibt der Schieber 21 zunächst in der beschriebenen Stellung, da der Halteknopf 211 ja in der Oeffnung 212 festgehalten wird. Der Druckknopf 2 hingegen bewegt sich abwärts. Beim Erreichen der ersten Relativposition (Fig. 2) kann der Schieber 21 in die Nut 22 des Druckknopfs 2 hineinrutschen und so kann die Blattfeder 210 den Schieber 21 in Richtung des Pfeils V (Fig. 11) bewegen. Dadurch wird der Halteknopf 211 durch die Blattfeder 210 noch weiter durch die Oeffnung 212 herausgedrückt. In diesem Zustand (Fig. 12, Fig. 13) kann die Vorratskammer 4 nicht mehr von der Rückstellfeder 43 (Fig. 2) in den Ausgangszustand (zweite Relativposition) zurückgestellt werden, da der Druckknopf 2 über den Schieber 21 fest eingerastet ist.

Erst wenn der Benutzer von aussen gegen den Halteknopf 211 in Richtung des Pfeils E drückt, wird der Schieber 21 aus der Nut 22 im Druckknopf 2 und somit aus seiner eingerasteten Stellung heraus bewegt und so die Arretierung gelöst. Nun kann die Vorratskammer von der Rückstellfeder 43 wieder in den Ausgangszustand (zweite Relativposition von Dosierstift 5 und Vorratskammer 4) zurückgestellt werden. Durch diese Art der Arretierung werden Mehrfach-Dosierungen verhindert.

Der bis hierher beschriebene Inhalator kann, wie bereits erwähnt, am Lufteinlass 3 mit einem Rückschlagventil versehen sein. Alternativ könnte das Rückschlagventil R auch im Saugansatz vorgesehen sein. Dieses Rückschlagventil R kann beispielsweise als Klappe ausgeführt sein, wie in Fig. 1 strichliert angedeutet, und bewirkt, dass beim Hineinblasen in den Inhalator durch den Auslass 10, z.B. bei versehentlichem Husten des Benutzers in den Auslass 10 hinein, der Lufteinlass 3 verschlossen ist. Das ist insofern besonders vorteilhaft, als beispielsweise nach erfolgter Dosierung des Pulvers und nach erfolgtem Rückstellen der Vorratskammer 4 in die zweite Relativposition, das Pulver nicht durch den Lufteinlass 3 hindurch aus dem Inhalator herausgeblasen werden kann. Hustet ein Benutzer nach erfolgter Dosierung also versehentlich in den Inhalator hinein, so geht kein Pulver verloren und er kann erneut ansaugen, ohne vorher neu dosieren zu müssen.

Weiterhin kann der Inhalator noch mit einem rücksetzbaren Zähler 7 ausgestattet sein (Fig. 1), der bei jedem Dosiervorgang inkrementiert wird. Beispielsweise wird der Zähler 7 bei der Abwärtsbewegung der Vorratskammer 4 inkrementiert. Ein solcher Zähler 7 ist besonders vorteilhaft, da die in der Vorratskammer 4 befindliche Kapsel K eine bestimmte Anzahl an Dosierungen des Pulvers enthält. Beim Einbringen einer neuen Kapsel K kann dann der Benutzer den Zähler 7 zurücksetzen, so dass er jederzeit erkennen kann, wieviele Dosierungen er der Kapsel K schon entnommen hat, und kann so rechtzeitig eine neue Kapsel K in die Vorratskammer 4 einlegen bzw. gegebenenfalls auch den Inhalator ersetzen.

Um den Innenraum des Inhalators bzw. den Luftweg zu trocknen bzw. trocken zu halten, ist in der Schutzkappe 11 ein Trockenmittel 12 vorgesehen, das beispielsweise als Silica-Gel ausgebildet sein kann.

Ein solcher Inhalator ist, wie bereits angedeutet, besonders geeignet für festkörperartige Stoffe oder Stoffgemische mit antiasthmatischer Wirkung, speziell auch zur Inhalation einer Mischung aus Lactose und Formoterol, das z.B. in Form seines Salzes Formoterol fumarat vorliegen kann, dessen Bezeichnung nach der IUPAC-Nomenklatur "(±)-2'-Hydroxy-5'-[(RS)-1-hydroxy-2-[[(RS)-p-methoxy-$\alpha$-methylphenethyl]-amino]-ethyl]-formanilid·fumarat·dihydrat" lautet.

Zu dem beschriebenen Inhalator sind viele Varianten denkbar. Prinzipiell muss gemäss dem Erfindungsgedanken nicht unbedingt die Vorratskammer bewegbar und der Dosierstift ortsfest angeordnet sein, ebenso könnte es umgekehrt der Fall sein. Es muss nur gewährleistet sein, dass die Vorratskammer und der Dosierstift mit der Dosierkerbe relativ zueinander bewegbar sind. Weiterhin ist es auch denkbar, dass das Pulver bzw. die Festkörper direkt in die Vorratskammer eingelagert sind und nicht in einer separaten Kapsel, die in diese Vorratskammer eingebracht wird. Das Erschütterungselement (Knackfeder) kann selbstverständlich anders ausgeführt sein, ebenso wie die Arretierungseinrichtung. Das Trockenmittel kann ebenfalls etwas anderes sein als das erwähnte Silica-Gel.

In den Figuren 14 bis 30 sind abgewandelte Ausführungsbeispiele des Inhalators I dargestellt, bei denen vor allem die vorerwähnte Relativbewegung zwischen Vorratskammer 4 und Dosierstift 5 in besonders zweckmässiger Weise gestaltet ist. Die Teile dieses abgewandelten Inhalators I sind dabei zu einem erheblichen Teil mit denselben Bezugszahlen wie bei dem vorbeschriebenen Ausführungsbeispiel versehen. Dabei zeigt der Vergleich beispielsweise zwischen Fig.1 und 14, dass bei dem nachfolgend beschriebenen Ausführungsbeispiel der Inhalator zwar ebenfalls in zwei Gehäuseteile unterteilt ist, die Trennung aber unterhalb des Luftauslasses oder Mundstückes 10 erfolgt.

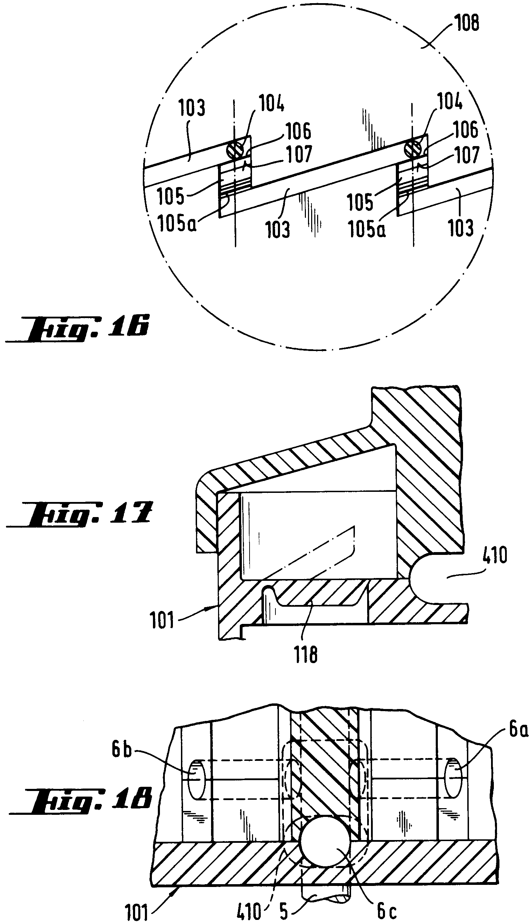

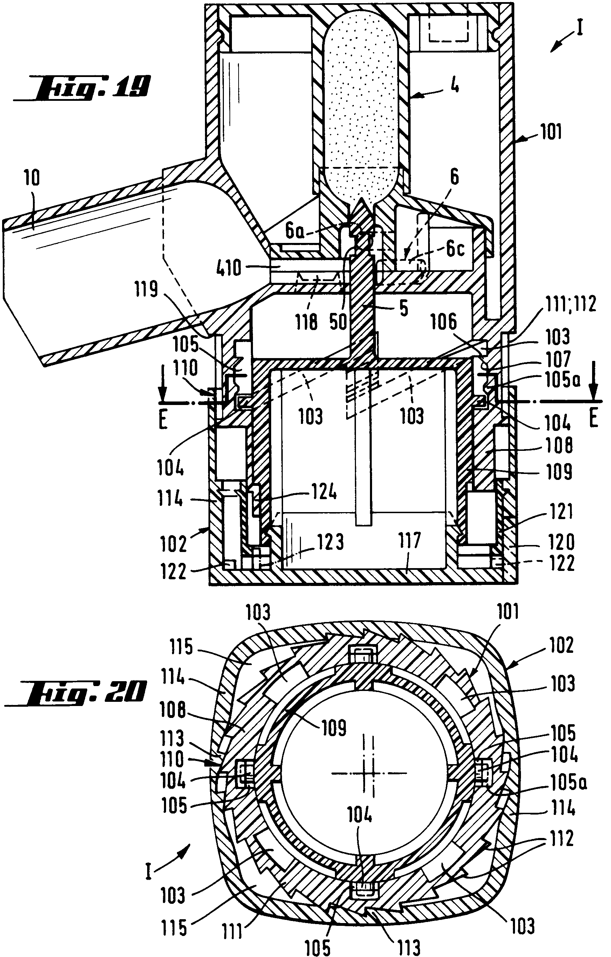

Um die Relativbewegung zwischen Vorratskammer 4 und Dosierstift 5 durchzuführen, ist bei den nachfolgend beschriebenen Ausführungsbeispielen vorgesehen, dass ein die Vorratskammer 4 aufweisender Gehäuseteil 101 und der den Dosierstift 5 tragende Gerätekörper oder Gehäuseteil 102 dadurch aus einer ersten zusammengeschobenen Position beispielsweise gemäss Fig. 14 in eine zweite auseinandergezogenen Position beispielsweise gemäss Fig. 19 relativ zueinander verschiebbar sind, dass sie relativ zueinander verdrehbar sind und schräge Führungsflächen oder Schräglächen 103 an dem Gehäuseteil 101 und daran geführte Gegenstücke 104, die an dem Gerätekörper 102 angebracht sind, die erwähnte Drehbewegung in eine Axialbewegung umsetzten. Es sei an dieser Stelle allerdings noch einmal darauf hingewiesen, dass ebenso die Schrägflächen 103 an der Aussenseite der Wandung 109, also am Gerätekörper 102, vorgesehen sein können, und die Gegenstücke 104 dementsprechend am Gehäuseteil 101. Eine Schrägfläche 103 ist in Fig. 16 als Abwicklung der Ansicht A der Fig. 14 dargestellt.

Dabei weist das Gehäuseteil 101 im Ausführungsbeispiel mehrere, nämlich jeweils vier an seinem Umfang verteilt angeordnete Schräglächen 103 gleicher Steigung und Länge und der relativ dazu verdrehbare Gehäuseteil 102 die entsprechende Anzahl von damit zusammenwirkenden Vorsprüngen oder Gegenstücken 104 auf.

Die vier Schrägflächen 103, die zweckmässigerweise als Nuten ausgebildet sind, um die vorsprungartigen Gegenstücke 104 gut zwangsweise führen zu können, erstrecken sich dabei über nahezu 90 Grad des Umfanges, liegen auf gleicher Höhe und steigen in gleicher Richtung an. Am Ende der Schrägflächen 103 ist jeweils eine in vertikaler Richtung, also parallel zur Längsmittelachse des Inhalators I orientierte Durchgangsnut 105 für die vorsprungsartigen Gegnstücke 104 vorgesehen, so dass nach einer Verdrehung und dem Entlanggleiten der Gegenstücke 104 oder Vorsprünge an den Schräglächen 103 mit der daraus resultierenden Axialverstellung des Dosierstiftes 5 der Gehäuseteil 101 mit Vorratskammer 4 und das als Führungsstift dienende Gegenstück 104 in axialer Richtung wieder zusammenschiebbar, also in die Ausgangslage gemäss Fig.14 zurückstellbar sind. Aus dieser Ausgangslage kann dann die nächste Dosier-Drehbewegung in der gleichen Drehrichtung wie die vorhergehende durchgeführt werden. Es ergibt sich also für den Benutzer eine sehr einfache Handhabung, wobei er in vorteilhafter Weise eine relativ leicht und sicher durchzuführende Drehbewegung machen kann, die auch einem älteren Menschen oder einem Kind leichter fällt, als wenn eine relativ kurze Axialverschiebung gegen den Anfangs-Reibwiderstand durchgeführt werden muss.

Im Verlauf der axialen Rückführnuten 105 ist gemäss Fig. 14, 16, 19 22 und 29 wenigstens ein sägezahnartig ausgebildeter Vorsprung 106 angeordnet, dessen steil abfallende Seite in Flucht mit der jeweiligen wirksamen Schrägfläche 103 für das Gegenstück oder den Vorsprung 104 angeordnet ist. Das Gegenstück 104 kann über die ansteigende äussere Schrägseite 107 des Vorsprungs 106 in axialer Richtung verschoben werden, so dass es in Ausgangslage der nächsten Dosierbewegung hinter der steil abfallenden Seite des Vorsprunges 106 angeordnet und von dieser steil abfallenden Seite während der Drehbewegung in die Schrägfläche 103 übergeleitet wird. Dadurch ist sichergestellt, dass der Benutzer eine Dosierung immer nur über eine Drehbewegung und nicht etwa über eine Axialbewegung durch Verschieben der Gegenstücke 104 entlang der Nut 105 durchführen kann und muss. Ferner ist im Verlauf der axialen Rückführnut 105 noch ein kleiner Vorsprung oder Nocken 105a vorgesehen, der ein kraftloses und eventuell ungewolltes Zusammendrükken des Inhalators verhindert.

Die Schräglächen 103 beziehungsweise die sie enthaltenden Nuten entsprechen jeweils einem Bruchteil eines Gewindeganges und sind im Ausführungsbeispiel an der Innenseite eines Wandstückes 108 des Gehäuseteiles 101 angeordnet, welches Wandstück 108 sich von dem Gehäuseteil 101 etwa axial nach der der Vorratskammer 4 entgegengesetzten Seite - in den dargestellten Figuren nach unten - erstereckt. An einer entsprechenden, parallel zu dieser Wand 108 angeordneten Wandung 109 des Gerätekörpers 102, welcher im Ausführungsbeispiel innerhalb des Wandstückes 108 angeordnet ist, stehen die Gegenstücke 104 radial nach aussen vor und greifen in die nutenförmigen Schräglächen 103 ein.

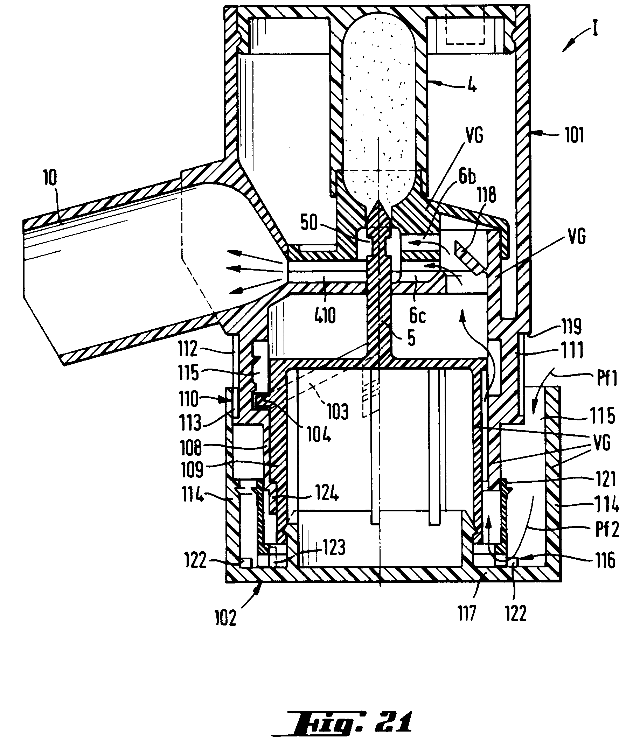

Um die Knackfeder 20 des Ausführungsbeispieles gemäss den Figuren 1 bis 13 zu ersetzen, dennoch aber bei der Dosierbewegung Erschütterungen zum Schütteln der Festkörper zu erzeugen und gleichzeitig in vorteilhafter Weise eine der Dosierbewegung entgegengesetzte Drehbewegung auszuschliessen, ist an Berührstellen zwischen den relativ zueinander verdrehbaren Gehäuseteilen 101 und 102 ein als Ratsche wirkendes Gesperre 110 angeordnet. Man erkennt dies vor allem in Fig.20, in der ausserdem die Rückführnuten 105 für die Gegenstücke 104 erkennbar sind.

Ausserdem sind in Fig.20 durch die horizontale Schnittführung dieser Darstellung gemäss der Schnittlinie E-E Fig.19 die Schrägflächen 103 teilweise angeschnitten und somit jeweils segmentförmig erkennbar.

Das Gesperre 110 weist in an sich üblicher Weise ein Gesperrerad 111 mit Sägezähnen 112 an dem Gehäuseteil 101 und zwar dem Wandstück 108 auf und wirkt mit jeweils in Umfansrichtung gegeneinander versetzten Gegenzähnen 113 oder Gruppen von solchen Gegenzähnen 113 zusammen, die an dem anderen Gehäuseteil oder Gerätekörper 102 an der Innenseite einer Aussenwandung 114 vorgesehen sind, welche gemäss Fig.20 keine kreisrunde Kontur hat, sondern gegnüber der kreisrunden Form des Gesperrerades 111 jeweils Eckräume 115 freilässt, so dass die erwähnten Gruppen von Gegenzähnen in der erwähnten Weise gegeneinander versetzt sein können, so dass eine Verdrehung des Gesperrerades 111 schon um weniger als eine Zahnteilung wieder zu einer Blockierung der Gegendrehrichtung führt. Die erwähnten Eckräume 115 werden nachfolgend noch im Zusammenhang mit der Luftzufuhr erwähnt.

Vor allem bei gleichzeitiger Betrachtung der Figuren 20 und 19 erkennt man, dass das Gesperrerad 111 im Grunde ein Teil der Wandung 108 des Gehäuseteiles 101 ist, der über einen kurzen axialen Bereich die Sägezähne 112 hat, die nach aussen vorstehen und in die Gegenzähne 113 der Aussenwandung 114 eingreifen, welche dabei eine grössere axiale Länge haben, um die axiale Relativbewegung zwischen Gehäuseteil 101 und Gerätekörper 102 zu berücksichtigen. Dabei erstrecken sich die Zähne 112, die Zahnlücken und die Gegenzähne 113 geradlinig in axialer Richtung, um die erwähnte axiale Relativbewegung, in welche die Drehbewegung ja umgesetzt wird, zu ermöglichen und auch zu führen.

Der den Dosierstift 5 aufweisende Gerätekörper 102 oder Gehäuseteil hat also bereichsweise eine Doppelwandung, deren innere Wandung 109 die mit den Schräglächen 103 an dem anderen Gehäuseteil 101 zusammewirkenden, radial nach aussen stehenden Gegenstücke 104 beträgt, während die äussere Wandung oder Aussenwandung 114 das die Schrägflächen 103 aufweisende Wandstück 108 des anderen Gehäuseteiles 101 aussen umgreift und die Gegenzähne 113 für das an der Aussenseite des die Vorkaminer 4 enthaltenden Gehäuseteiles 101 beziehungsweise der Wandung 108 angeordnete Gesperrerad 111 enthält. Dies ergibt die in Fig. 14, 19, 21, 22 und 29 erkennbare kompakte Bauweise und Verbindung des Gehäuseteiles 101 mit dem Gerätekörper 102, obwohl diese beiden Teile nicht nur in axialer Richtung, sondern auch in Drehrichtung gegeneinander verstellbar sind.

Um die erwähnte Drehbewegung ungehindert durchführen zu können, sind die innere Wandung 109 des die Dosiernadel 5 aufweisenden Gehäuseteiles 102 und die die schrägen Führungsflächen 103 aufweisende Wand 108 des die Vorratskammer 4 aufweisenden Gehäuseteiles 101 im Querschnitt kreisrund, während die äussere Wandung 114 des ersteren Gehäuseteiles 102 - wie bereits erwähnt - zumindest bereichsweise von dieser Kreisform abweicht, so dass die Eckräume oder Eckbereiche 115 gebildet sind. Insbesondere bei verdrehten und/oder axial auseinandergeschobenen Gehäuseteilen 101 und 102 kann gemäss Fig.21, in der einige Teile VG verdreht dargestellt sind, die angesaugte Luft gemäss den Pfeilen Pf 1 und Pf 2 in Fig.21 über diese dann offenen Eckbereiche 115 angesaugt und über Durchbrüche 116 nahe dem Boden 117 des Gerätekörpers 102 sowie wenigstens über eine Rückschlagkappe 118 - gemäss Fig. 15 zwei Rückschlagklappen 118 - zu den Luftkanälen 6a, 6b und 6c und dem Dosierstift 5 geführt werden.

Dadurch ist es gleichzeitig möglich, dass die Stirnseite der Aussenwandung 114 des die Dosiernadel enthaltenden Gerätekörpers 102 in zusammengeschobener Position gegen eine Anschlagfläche 119 des anderen Gehäuseteiles 101 mit übereinstimmender umlaufender Kontur anliegt und in dieser Position der Luftweg abgeschnitten und das Innere des Inhalators I luftdicht verschlossen ist. Die Absperrung der Luft gegenüber den Luftkanälen 6a, 6b und 6c und dem Dosierbereich ist also nicht mehr ausschliesslich von der Rückstellkraft der Rückstellklappe oder des Rückschlagventiles R abhängig, sondern in zusammengeschobener Position wird der Luftweg zusätzlich dicht verschlossen.

Die erfindungsgemäss in die axiale Dosierbewegung umsetzbare Drehbewegung erlaubt ausserdem eine Kontrolle und Anzeige der schon durchgeführten Dosierungen, statt einen Zähler 7 zu verwenden. Bei den Ausführungsbeispielen nach Fig. 14 bis 30 enthält der eine Gehäuseteil einen relativ zu einem Anzeigefenster 120 verdrehbaren Anzeigering 121, der durch die Relativ-Verdrehung der beiden Gehäuseteile 101 und 102 zueinander beim Dosieren jeweils um einen kleinen Winkelbetrag gleicher Grösse und - im Hinblick auf die immer in gleicher Richtung durchzuführende Dosier-Drehbewegung - gleicher Richtung weiterbewegbar ist. Die genaue Gestaltung und Anordnung einer solchen Anzeigevorrichtung ist in den Figuren 22 bis 28 dargestellt.

In diesen Figuren erkennt man, dass der Anzeigering 121 als Zahnrad ausgebildet ist, welches exzentirisch zu einem Hohl-Zahnrad 122 mit einer Hohlverzahnung oder Innenverzahnung in dem die Dosiernadel 5 aufweisenden Gehäuseteil oder Gerätekörper 102 gelagert ist und mit seiner Aussenverzahnung in die Innenverzahnung dieses Hohlrades 122 passt, welches Hohlrad mit dem erwähnten Gehäuseteil 102 fest verbunden ist. Die Zähnezahl dieses inneren, als Anzeigering 121 dienenden Zahnrades weicht dabei gegenüber der Zähnezahl des Hohlrades 122 so wenig ab, ist also nur um eine geringe Anzahl kleiner, dass das Innenzahnrad gegenüber dem Hohl-Zahnrad 122 um etwa eine einzige Umdrehung oder einen Bruchteil davon verdrehbar ist, wenn eine vorbestimmte Anzahl von zum Beispiel 100 bis 200 Dosierungen durchgeführt ist. In Figur 23 bis 28 ist angedeutet, dass jeweils der Aussenmantel des Anzeigeringes 121 an dem Anzeigefenster 120 von aussen her sichtbar sind, insbesondere, wenn das Anzeigefenster durchscheinend oder durchsichtig und der Anzeigering 121 in geeigneter Weise gefärbt sind. Dabei kann die Farbe über den Umfang des Anzeigeringes 121 wechseln, um den Dosierfortschritt beziehungsweise den Fortschritt der Entleerung der Vorratskammer 4 zu verdeutlichen.

Die Art der Anzeige durch das mehrmalige Verdrehen des Gerätekörpers 102 gegenüber dem Gehäuseteil 101 um eine viertel Umdrehung und die Weiterbewegung des Anzeigeringes 121 ist dabei in den Figuren 24 bis 28 dadurch verdeutlicht, dass einer der Zähne des Anzeigeringes 121 markiert ist. Gleichzeitig ist in diesen Figuren noch dargestellt, dass der Anzeigering 121 und damit die Dosierbewegung nach Erreichen der vorgesehenen Anzahl von Dosierungen blockierbar ist.

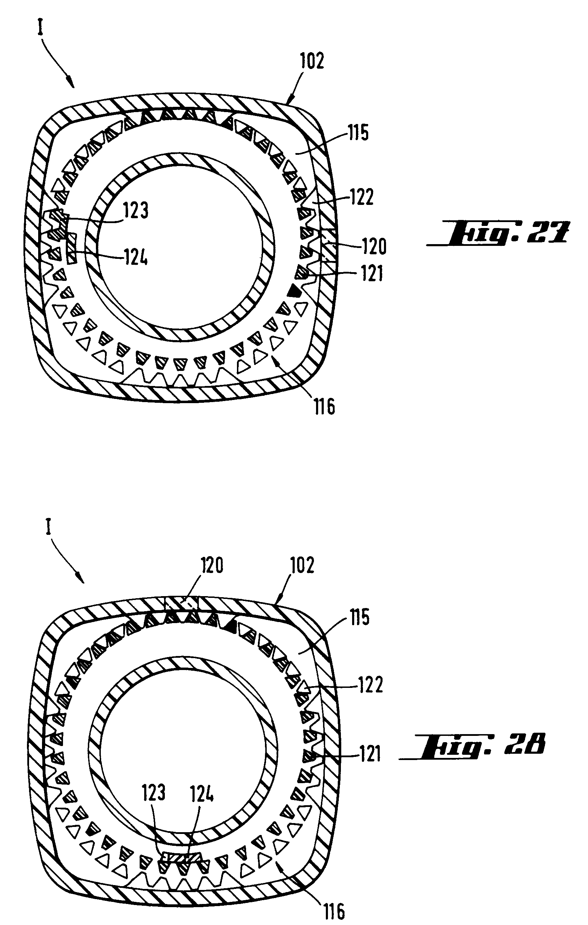

Während in Fig.24 der Anlieferungszustand des Inhalators I dargestellt ist und sich der markierte Zahn an der einen Seite des Anzeigefensters 120 befindet, zeigt Fig.25 eine erste Dosierung, bei welcher der das Anzeigefenster 120 aufweisende Gerätekörper 102 um 90 Grad verdreht wurde. Entsprechend wird der Anzeigering 121 über die Verzahnung mitgenommen. Danach erfolgt die axiale Rückstellbewegung und weitere Dosierungen, wobei Fig.26 beispielsweise die Position nach der vierten Dosierung andeutet. Der markierte Zahn hat nun etwa die Breite des Anzeigefensters 120 durchlaufen.

Wesentlich ist dabei die gleichzeitig sich vorbereitende, schon erwähnte Blockierung der Dosierbewegung nach einer vorgesehenen Anzahl von Dosierungen, in diesem Falle von beispielsweise 200 Dosierungen.

Diese Blockierung erfolgt dabei mit Hilfe des Anzeigeringes 121 auf folgende Weise

Das als Anzeigering 121 dienende Zahnrad weist auf seiner den Zähnen abgewandten Innenseite einen Anschlag 123 auf, der mit einem Gegenanschlag 124 des relativ zu ihm verdrehbaren Gehäuseteiles 101 in dem Sinne zusammenwirkt, dass die beiden Anschläge 123 und 124 in Ausgangslage gemäss Fig.24 einander nahe sind oder sich sogar berühren und durch die Dosierbewegungen gemäss den Figuren 25 bis 27 allmählich in Umfangsrichtung zunächst auseinander und dann wieder aufeinanderzu bewegbar sind, wobei durch die gleichzeitige Axialverstellung diese beiden Anschläge 123 und 124 jeweils in zwei gegeneinander höhenversetzte Ebenen gelangen und durch die Rückbewegung wieder in eine übereinstimmende Ebene oder etwa auf gleiche Höhe zu liegen kommen. Gleichzeitig sorgt dabei auch die Exzentrizität dafür, dass die beiden Anschläge auch nach einer relativ grossen Anzahl von Dosierungen, z.B. nach 197 Dosierungen (bei der letzten Dosierung), gemäss Fig.27 noch nicht miteinander kollidieren.

Fig.28 zeigt, dass die beiden Anschläge 123 und 124 nach etwa einer Umdrehung des Anzeigeringes 121 und einem letzten Dosiervorgang in den beiden Ebenen derart übereinanderliegen, dass die axiale Rückverstellung in die Ausgangslage des Inhalators gemäss Fig. 14 und damit ein erneuter, aus dieser Lage beginnender Dosiervorgang gesperrt sind (Blockierung ist wirksam). Die zum Dosieren dienenede Drehbewegung kann also nicht nur gleichzeitig in vorteilhafter Weise für die Verdrehung eines Anzeigeringes nutzbar gemacht werden, sondern dieser kann mit Hilfe der Anschläge 123 und 124 eine zusätzliche Funktion erhalten und zwar dazu dienen, nach einer vorgegebenen Anzahl von Dosiervorgängen den Inhalator I zu sperren. Dadruch kann verhindert werden, das bei einer weitgehenden Entleerung des Inhalators I der Benutzer weiterhin Dosiervorgänge durchführt, aber keine ausreichende Menge an Festkörpern mehr erhält.

In den Figuren 29 und 30 sind Ausführungsbeispiele des erfindungsgemässen Inhalators I dargestellt, bei denen die vorerwähnten Merkmale und Massnahmen ebenfalls erfüllt sind, zusätzlich aber in der Vorratskammer 4 auf der dem Dosierstift 5 entgegengesetzten Seite ein von einer schwachen Feder 125, in diesem Falle einer Schraubfeder, beaufschlagter, der Abnahme des Inhaltes der Vorratskammer 4 folgender Kolben 126 angeordnet ist. Die Federkraft ist dabei so gering, dass eine Kompression der Festkörper nicht stattfindet, wobei die Federkraft gleichzeitig aber so stark ist, dass sie den Kolben 126 auch gegen die Schwerkraft der Festkörper und seines eigenen Gewichtes der Abnahme des Kammervolumens folgend verschieben kann. Dadurch wird der gesamte Inhalator I in praktisch jeder beliebigen Lage verwendbar, das heisst ein Benutzer kann ihn auch im Liegen benutzen, ohne eine schwierige Lage einnehmen zu müssen. Es bleibt trotzdem immer sichergestellt, dass jede Dosierbewegung dazu führt, die der Dosierkerbe 50 entsprechende Menge an Festkörpern inhalieren zu können.

Gleichzeitig wird dabei durch diese Figuren und vor allem Fig.30 deutlich, dass die Vorratskammer 4 innerhalb des Gehäuseteiles 101 lösbar und auswechselbar angeordnet sein kann und/oder einen lösbaren Deckel 127 haben kann. Somit braucht nach der Entleerung der Vorratskammer 4 nicht der gesamte Inhalator I, sondern nur die Vorratskammer ersetzt zu werden oder sie kann eventuell sogar nachgefüllt werden. Entsprechend gering ist der Anfall an Abfall.

Fig.30 verdeutlicht dabei noch, dass die auswechselbar in dem Gehäuseteil 101 angeordnete Vorratskammer 4 in Ausgangsstellung vor der Montage einen durchstossbaren, ausstossbaren und/oder über Sollbruchstellen 128 dicht befestigten Verschluss 129 des Durchtrittskanales 400 für den Dosierstift 5 hat, welcher Verschluss 129 durch das Einfügen der Vorratskammer 4 in ihr Gehäuseteil 101 mittels des Dosierstiftes 5 automatisch eindrückbar und öffenbar ist. Somit ist das Ersetzen einer weitgehend entleerten Dosierkammer 4 durch eine gefüllte Dosierkammer 4 sehr einfach, weil gleichzeitig durch ihr Einschieben in Gebrauchsstellung auch schon die Verbindung mit dem Luftweg hergestellt wird und der Dosierstift 5 in seine erste Dosierstellung gelangt.

Dabei kann der durch die Montage öffenbare Verschluss 129 der Vorratskammer 4 gegebenenfalls in das Innere der Vorratskammer 4 aufschwenkbar sein, also noch mit der Wandung der Vorratskammer 4 verbunden bleiben, und sein Durchmesser ist dabei zweckmässigerweise grösser als die axiale Erstreckung der Dosierkerbe 50, so dass diese problemlos an dem nach innen geschwenkten Verschluss 129 vorbeigleiten kann.

Wie bereits erwähnt, hat der erfindungsgemässe Inhalator mehrere Vorteile. Eine sichere, schnelle, zuverlässige und vollständige Befüllung der Dosierkerbe ist gewährleistet, so dass die dosierte Menge an Festkörpern eine gute Konstanz aufweist und so ein in Atemnot geratener Benutzer schnell und zuverlässig eine bestimmte Menge an linderndem Pulver inhalieren kann. Weiterhin eignet sich der Inhalator speziell auch zur Inhalation von feinkörnigen und daher schlecht liessfähigen Pulvern. Ausserdem wird das Eindringen von Feuchtigkeit in den Inhalator und damit eine Klümpchenbildung des Pulvers vermieden. Weiterhin kann bei dem erfindungsgemässen Inhalator das Pulver, wie beschrieben, in einer Kapsel gelagert werden, so dass auch solche Pulver verwendet werden können, deren pharmazeutische Wirkung durch Dauerkontakt mit der Luft (direkte Lagerung in der Vorratskammer) nachlässt (siehe z.B. Konstruktion gemäss Fig.30). Der Inhalator ist einfach demontierbar, reinigbar, leicht zu bedienen und ist auch nicht als Wegwerf-Inhalator ausgebildet. Besonders geeignet ist er für die Inhalation von antiasthmatisch wirksamen Stoffen und Stoffgemischen und hier ganz speziell für ein Stoffgemisch aus Formoterol und Lactose. Vor allem die leichte Bedienbarkeit ergibt sich in erhöhtem Masse, wenn die axiale Dosierbewegung durch eine Teil-Verdrehung der beiden Gehäuseteile 101 und 102 gegeneinander bewirkt wird, weil eine solche Verdrehung für den Benutzer wesentlich sicherer durchführbar ist, als eine relativ kurze, durch Reibkräfte erschwerte reine Axialverschiebung.

Der Inhalator I zum Einbringen einer dosierten Menge von Festkörpern, zum Beispiel pharmazeutisch wirksamen Pulvern, in einen von einem Benutzer angesaugten Luftstrom umfasst eine Vorratskammer 4, in die ein Dosierstift 5 hineinragt. Der Dosierstift 5 ist mit einer Dosierkerbe 50 versehen, welche die Menge an Festkörpern festlegt, die dem Luftstrom beigemischt wird. Die Vorratskammer 4 und der Dosierstift 5 sind relativ zueinander so bewegbar, dass sich in einer ersten Relativposition von Dosierstift 5 und Vorratskammer 4 die Dosierkerbe 50 des Dosierstiftes 5 in der Vorratskammer 4 befindet, wo sie mit Festkörpern befüllt wird, und in einer zweiten Relativstellung sich im Luftkanal befindet, wo die Festkörper dem Luftstrom beigemischt werden. Die axiale Relativbewegung kann dabei durch eine Verdrehung zweier Gehäuseteile 101, 102 über Schrägflächen 103 bewirkt werden.

## Patentansprüche

1. Inhalator zum Einbringen einer dosierten Menge von Festkörpern, insbesondere pharmazeubsch wirksamen festen Stoffen oder Stoffgemischen, vorzugsweise in Pulverform, in einen von einem Benutzer angesaugten Luftstrom, der nach dem Einbringen der Festkörper als Festkörper-Luft-Gemisch in die Atemwege des Benutzers gelangt, mit einem Lufteinlass (3) für die angesaugte Luft, mit einem Luftkanal (6), der den Lufteinlass (3) mit einem Auslass (10) beziehungsweise einem Mundstück verbindet, mit einer Vorratskammer (4) für die Festkörper, und mit einem Dosierstift (5), der mit Hilfe einer an ihm vorgesehenen Dosierkerbe (50) die Menge an Festkörpern festlegt und in den Luftstrom einbringt, dadurch gekennzeichnet, dass der Dosierstift (5) durch eine Oeffnung (400) der Vorratskammer (4) hindurch in Richtung seiner Längsachse in die Vorratskammer (4) hineinragt, und dass die Vorratskammer (4) und der Dosierstift (5) relativ zueinander in Richtung der Längsachse des Dosierstifts (5) so bewegbar sind, dass sich die Dosierkerbe (50) des Dosierstifts in einer ersten Relativposition in der Vorratskammer (4) und in einer zweiten Relativposition im Luftkanal befindet.

2. Inhalator nach Anspruch 1, dadurch gekennzeichnet, dass der Dosierstift (5) in Bezug auf den Gerätekörper ortsfest angeordnet ist, und dass die Vorratskammer (4) gegen den Dosierstift (5) bewegbar ausgebildet ist.

3. Inhalator nach Anspruch 2, dadurch gekennzeichnet, dass er ein separates Betätigungsorgan (2) zum Be-

EP 0 505 321 A2

wegen der Vorratskammer (4) umfasst, welches so ausgebildet ist, dass es bei Betätigung die Vorratskammer (4) gegen die Kraft einer Rückstellfeder (43) in die erste Relativposition zum Dosierstift (5) bewegt und dass die Wand der Vorratskammer (4) mit einem Wandfortsatz (41) versehen ist, der in dieser ersten Relativposition den Luftkanal verschliesst, und dass in dem Wandfortsatz eine Oeffnung (410) vorgesehen ist, die nach dem Rückstellen der Vorratskammer (4) in die zweite Relativposition den Luftkanal wieder freigibt.

4. Inhalator nach Anspruch 3, dadurch gekennzeichnet, dass das Betätigungsorgan (2) mit einem Erschütterungselement (20) versehen ist, welches nach Erreichen der ersten Relativposition von Dosierstift (5) und Vorratskammer (4) die Vorratskammer (4) erschüttert.

5. Inhalator nach Anspruch 4, dadurch gekennzeichnet, dass das Erschütterungselement als Knackfeder (20) ausgebildet ist.

6. Inhalator nach einem der Ansprüche 1 bis 5, dadurch gekennzeichnet, dass er mit einer Arretierungseinrichtung (21,210,211,212) versehen ist, welche die Vorratskammer (4) beziehungsweise den Dosierstift (5) in der ersten Relativposition zueinander festhält, und dass ein Entriegelungselement (211) zur Lösung der Arretierung vorgesehen ist.

7. Inhalator nach einem der Ansprüche 1 bis 6, dadurch gekennzeichnet, dass er einen gesonderten Saugansatz (1) aufweist, in welchem sich der Querschnitt des Luftkanals zum Auslass ( 10) hin vergrössert.

8. Inhalator nach Anspruch 7, dadurch gekennzeichnet, dass er eine separate Verschlusskappe (11) umfasst, die auf den Saugansatz (1) aufsetzbar ist.

9. Inhalator nach Anspruch 8, dadurch gekennzeichnet, dass in der Verschlusskappe (11) ein Trockenmittel (12) vorgesehen ist.

10. Inhalator nach einem der Ansprüche 1 bis 9, dadurch gekennzeichnet, dass am Lufteinlass (3) ein Rückschlagventil vorgesehen ist.

11. Inhalator nach einem der Ansprüche 1 bis 10, dadurch gekennzeichnet, dass er mit einem rücksetzbaren Zähler (7) versehen ist, der beim Erreichen der ersten Relativposition von Vorratskammer (4) und Dosierstift (5) inkrementiert wird.

12. Inhalator nach einem der Ansprüche 1 bis 11, dadurch gekennzeichnet, dass die Festkörper ein antiasthmatisch wirksamer Stoff oder ein antiasthmatisch wirksames Stoffgemisch sind.

13. Inhalator nach Anspruch 12, dadurch gekennzeichnet, dass der antiasthmatisch wirksame Stoff bzw. das antiasthmatisch wirksame Stoffgemisch eine Mischung aus Formoterol und Lactose ist.

14. Inhalator insbesondere nach einem der vorstehenden Ansprüche, dadurch gekennzeichnet, dass ein die Vorratskammer (4) aufweisender Gehäuseteil (101) und der den Dosierstift (5) tragende Gerätekörper (102) dadurch aus einer ersten zusammengeschobenen Position in eine zweite auseinandergezogene Position relativ zueinander verschiebbar sind, dass sie relativ zueinander verdrehbar sind und wenigstens eine Schrägfläche (103) and dem Gerätekörper (102) und/oder dem Gehäuseteil (101) und ein daran geführtes Gegenstück ( 104) die Drehbewegung in die Axialbewegung umsetzt.

15. Inhalator nach Anspruch 14, dadurch gekennzeichnet, dass der Gehäuseteil oder Gerätekörper (102) mehrere an seinem Umfang verteilt angeordnete Schrägflächen (103) gleicher Steigung und Länge und der relativ dazu verdrehbare Körper oder Teil die entsprechende Anzahl von damit zusammenwirkenden Vorsprüngen oder Gegenstücken (104) aufweist.

16. Inhalator nach Anspruch 14 oder 15, dadurch gekennzeichnet, dass jeweils vier sich über nahezu 90 Grad erstreckende, auf gleicher Höhe liegende und in gleicher Richtung ansteigende Schrägflächen vorgesehen sind, die mit als Vorsprüngen ausgebildeten Gegenstücken (104) zusammenwirken, und dass am Ende der Schrägflächen (103) in vertikaler Richtung orientierte Durchgänge, Nuten ( 105) oder dergleichen für die Gegenstücke (104) vorgesehen sind, so dass nach einer Verdrehung und dem Entlanggleiten der Gegenstücke (104) an den Schrägflächen (103) mit der daraus resultierenden Axialverstellung des Dosierstiftes (5) der Gehäuseteil (101) mit Vorratskammer (4) und das Gegenstück (104) in axialer Richtung

14

zusammenschiebbar und in Ausgangslage zurückstellbar sind.

17. Inhalator nach einem der Ansprüche 14 bis 16, dadurch gekennzeichnet, dass im Verlauf der axialen Rückführnuten ( 105) wenigstens ein nockenartiger Vorsprung (105a) vorgesehen ist, über den hinweg das Gegenstück (104) verschiebbar ist, dass im Verlauf der axialen Rückführnuten (105) weiterhin wenigstens ein sägezahnarig ausgebildeter Vorsprung (106) angeordnet ist, dessen steil abfallende Seite in Flucht mit der wirksamen Schrägfläche (103) für das Gegenstück (104) angeordnet ist, und dass das Gegenstück (104) über die ansteigende Schrägseite (107) dieses Vorsprunges (106) in axialer Richtung verschiebbar und in Ausgangslage der nächsten Dosierbewegung hinter der steil abfallenden Seite angeordnet ist.

18. Inhalator nach einem der Ansprüche 14 bis 17, dadurch gekennzeichnet, dass die Schrägflächen ( 103) vorzugsweise nutenförmig sind und Bruchteilen eines Gewindeganges entsprechen und insbesondere an der Innenseite eines Wandstückes (108) des Gehäuseteiles (101) angeordnet sind.

19. Inhalator nach einem der Ansprüche 14 bis 18, dadurch gekennzeichnet, dass an Berührungsstellen zwischen den relativ zueinander verdrehbaren Gehäuseteilen (101, 102) ein Gesperre ( 110), eine Ratsche oder dergleichen angeordnet ist, das ein gegensinniges Verdrehen der Gehäuseteile entgegen der Dosierbewegung sperrt.

20. Inhalator nach einem der Ansprüche 14 bis 19, dadurch gekennzeichnet, dass das Gesperre (110) ein Gesperrerad (111) mit Sägezähnen (112) an dem einen Gehäuseteil (101) und mit jeweils in Umfangsrichtung gegeneinander versetzten Gegenzähnen (113) oder Gruppen von Gegenzähnen (113) an dem anderen Gehäuseteil (102) oder Gerätekörper aufweist, wobei der Versatz der jeweils vorgesehenen Gegenzähne (113) in Umfangsrichtung gegeneinander derart gewählt ist, dass die Rastschritte des Gesperres kleiner als die Zahnteilung sind.

21. Inhalator nach Anspruch 19 oder 20, dadurch gekennzeichnet, dass sich die Zähne (112), die Zahnlücken und die Gegenzähne (113) geradlinig in axialer Richtung erstrecken.

22. Inhalator nach einem der Ansprüche 14 bis 21, dadurch gekennzeichnet, dass der den Dosierstift (5) aufweisende Gerätekörper (102) oder Gehäuseteil bereichsweise eine Doppelwandung hat, deren innere Wandung (109) die mit den Schrägflächen (103) an dem anderen Gehäuseteil (101) zusammenwirkenden Gegenstücke (104) trägt, die von dieser inneren Wandung radial nach aussen abstehen, und dass die äussere Wandung (114) das die Schrägflächen (103) aufweisende Wandstück (108) des anderen Gehäuseteiles (101) aussen umgreift und die Gegenzähne (113) für das an der Aussenseite des die Vorratskammer (4) enthaltenden Gehäuseteiles (101) angeordnete Gesperrerad (111) enthält.

23. Inhalator nach einem der Ansprüche 14 bis 22, dadurch gekennzeichnet, dass die innere Wandung (109) des die Dosiernadel (5) aufweisenden Gehäuseteiles (102) und die die Führungsflächen (103) aufweisende Wand (108) des die Vorratskammer (4) aufweisenden Gehäuseteiles (101) kreisrund sind und dass die äussere Wandung (114) des ersteren Gehäuseteiles (102) zumindest bereichsweise einen von dieser Kreisform abweichenden Querschnitt hat, so dass Eckbereiche ( 115) zwischen den Wandungen gebildet sind, und dass insbesondere bei verdrehten und/oder axial auseinandergeschobenen Gehäuseteilen (101, 102) die Luft über diese dann offenen Eckbereiche (115) angesaugt und über Durchbrüche (116) nahe dem Boden (117) des Gerätekörpers (102) sowie über wenigstens eine Rückschlagkappe zu den Luftkanälen (6a, 6b, 6c) und dem Dosierstift (5) geführt ist.

24. Inhalator nach einem der Ansprüche 14 bis 23, dadurch gekennzeichnet, dass die Stirnseite der Aussenwandung ( 114) des die Dosiernadel enthaltenden Gerätekörpers (102) in zusammengeschobener Position gegen eine Anschlagfläche (119) des anderen Gehäuseteiles (101) mit übereinstimmender umlaufender Kontur anliegt und in dieser Position der Luftweg abgeschnitten und das Innere des Inhalators (I) luftdicht verschlossen ist.

25. Inhalator nach einem der Ansprüche 14 bis 24, dadurch gekennzeichnet, dass der eine Gehäuseteil einen relativ zu einem Anzeigefenster (120) verdrehbaren Anzeigering (121) enthält, der durch die Relativ-Verdrehung der beiden Gehäuseteile zueinander beim Dosieren jeweils um einen kleinen Winkelbetrag gleicher Grösse und gleicher Richtung weiterbewegbar ist.

26. Inhalator nach einem der Ansprüche 14 bis 25, dadurch gekennzeichnet, dass der Anzeigering (121) als Zahnrad ausgebildet ist, welches exzentrisch zu einem Hohl-Zahnrad (122) oder zu einer Hohl-Verzahnung in dem die Dosiernadel (5) aufweisenden Gehäuseteil (102) gelagert ist und mit seiner Aussenverzahnung in die Innenverzahnung dieses Hohlrades (122) passt, wobei die Zähnezahl dieses inneren Zahnrades gegenüber der Zähnezahl des Hohlrades so wenig abweicht, dass das Innenzahnrad gegenüber dem Hohl-Zahnrad um etwa eine einzige Umdrehung oder einen Bruchteil davon verdrehbar ist, wenn eine vorbestimmte Anzahl von zum Beispiel 100 bis 200 Dosierungen durchgeführt ist.

27. Inhalator nach Anspruch 25 oder 26, dadurch gekennzeichnet, dass der Anzeigering ( 121 ) und damit die Dosierbewegung nach Erreichen der vorgesehenen Anzahl von Dosierungen blockierbar ist.

28. Inhalator nach einem der Ansprüche 14 bis 27, dadurch gekennzeichnet, dass das als Anzeigering (121) dienende Zahnrad einen Anschlag (123) aufweist, der mit einem Gegenanschlag (124) des relativ zu ihm verdrehbaren Gehäuseteiles (101) in dem Sinne zusammenwirkt, dass die beiden Anschläge in Ausgangslage einander nah sind oder sich berühren und durch die Dosierbewegungen allmählich in Umfangsrichtung auseinander bewegbar sind, wobei durch die gleichzeitige Axialverstellung diese beiden Anschläge (123, 124) jeweils in zwei gegeneinander höhenversetzten Ebenen zu liegen kommen und durch die Rückbewegung wieder in eine übereinstimmende Ebene gelangen, und dass die beiden Anschläge ( 123, 124) nach etwa einer Umdrehung des Anzeigeringes (121) und einem letzten Dosiervorgang in den beiden Ebenen derart übereinanderliegen, dass die axiale Rückverstellung in Ausgangslage und damit ein erneuter Dosiervorgang gesperrt sind.

29. Inhalator nach einem der Ansprüche 1 bis 28, dadurch gekennzeichnet, dass in der Vorratskammer (4) auf der dem Dosierstift (5) entgegengesetzten Seite ein von einer schwachen Feder ( 125) beaufschlagter, der Abnahme des Kammerinhaltes folgender Kolben (126) oder dergleichen angeordnet ist, wobei die Federkraft so gering ist, dass eine Kompression der Festkörper verhindert ist, wobei die Federkraft aber so stark ist, dass sie den Kolben (126) auch gegen die Schwerkraft der Festkörper und seines eigenen Gewichtes der Abnahme des Kammervolumens folgend verschiebt.

30. Inhalator nach einem der Ansprüche 1 bis 29, dadurch gekennzeichnet, dass die Vorratskammer (4) innerhalb des Gehäuseteiles ( 101 ) lösbar und auswechselbar angeordnet ist und/oder einen lösbaren Deckel ( 127) hat.

31. Inhalator nach einem der Ansprüche 1 bis 30, dadurch gekennzeichnet, dass die auswechselbar in dem Gehäuseteil (101) angeordnete Vorratskammer (4) in Ausgangsstellung vor der Montage einen durchstossbaren, ausstossbaren und/oder über Sollbruchstellen dicht befestigten Verschluss des Durchtrittskanales (400) für den Dosierstift (5) hat, welcher Verschluss (129) durch das Einfügen der Vorratskammer (4) in ihr Gehäuseteil (101) mittels des Dosierstifes (5) automatisch öffenbar ist.

**Fig. 1**

**Fig. 2**

Fig. 3

Fig. 4

Fig. 5

Fig. 6

Fig. 7

Fig. 8

*Fig. 9*

Fig. 10

Fig. 11

*Fig. 12*

*Fig. 13*

Fig. 14

Fig. 15

*Fig. 16*

*Fig. 17*

*Fig. 18*

*Fig. 19*

*Fig. 20*

_Fig. 21_

*Fig. 22*

*Fig. 23*

I 120 102

115

*Fig. 24*

122

121

124 123

116

I

122

121

120

123

124

102

115

116

*Fig. 25*

I

120 102

115

122

121

124 123

116

*Fig. 26*

I

102

115

123

124

122

120

121

116

_Fig. 27_

I

120

102

115

122

121

123 124

116

_Fig. 28_

_Fig. 29_

_Fig. 30_